# EUROPEAN PATENT APPLICATION

(11) **EP 4 806 903 A1**
(43) Date of publication of application: **16.09.2026**
(21) Application number: 24888089.0
(22) Date of filing: 08.11.2024
(51) Int. Cl.: C07K 7/06, A61K 31/704, A61K 51/08, A61K 49/00, A61K 49/14, A61K 47/64, A61K 47/66, A61K 39/395, A61P 35/00

(54) **WHOLE-PROCESS TARGETING POLYPEPTIDE, PHARMACEUTICAL COMPLEX THEREOF, DRUG DELIVERY SYSTEM AND USE THEREOF**

(30) Priority: 08.11.2023 CN 202311481108
(71) Applicant: Fudan University, Shanghai 200433 (CN)
(72) Inventor: LU, Weiyue, Shanghai 200433 (CN); ZHOU, Jianfen, Shanghai 200433 (CN); GUAN, Jiacheng, Shanghai 200433 (CN); DING, Yuan, Shanghai 200433 (CN); XIE, Cao, Shanghai 200433 (CN); MENG, Nana, Shanghai 200433 (CN); LU, Linwei, Shanghai 200433 (CN)
(74) Representative: Henderson, Helen Lee
(86) International application number: PCT/CN2024/130850
(87) International publication number: WO 2025/098478

(57) **Abstract**

Provided are a whole-process targeting polypeptide, a pharmaceutical complex thereof, a drug delivery system and the use thereof. The whole-process targeting polypeptide comprises pHA and a VAP which are covalently linked by means of a bridging structure; and the VAP is ^{L}VAP, a retro polypeptide of ^{L}VAP, ^{D}VAP or ^{S}VAP, the amino acid sequence of ^{L}VAP being as shown as SEQ ID NO: 1, ^{S}VAP being a D-configuration polypeptide of ^{L}VAP, ^{D}VAP being a D-configuration polypeptide of a retro polypeptide of ^{L}VAP, and the bridging structure being amino-monoethylene glycol-carboxylic acid, or a derivative thereof. The whole-process targeting polypeptide can cross the blood-brain barrier and target new blood vessels of tumors, can cross the blood-tumor barrier and target tumor vasculogenic mimicry-derived channels, tumor cells and stem cells thereof, and has better tumor cell affinity, anti-tumor treatment effect and in-vivo safety effect. In addition, when being combined with chemotherapeutic drugs, the pharmaceutical complex or the drug delivery system has a synergistic effect, thereby remarkably improving the anti-tumor pharmaceutical effect.

## Description

This application claims priority of Chinese patent application 2023114811082 filed on November 8, 2023, the contents of which are incorporated herein by its entirety.

### Technical field

The present invention belongs to the field of pharmaceuticals, in particular to a whole-process targeting peptide, a drug complex thereof, a drug delivery system thereof, and use thereof in tumor-targeted diagnosis and treatment.

### Background

Tumors are diseases that seriously threaten human life and health, with a mortality rate ranking first among all diseases. Among them, the incidence and mortality of primary brain tumors rank in the top 10 of tumor statistics in China, with gliomas accounting for approximately 45% of all primary brain tumors. The median survival time for patients with gliomas is less than 16 months, making them extremely dangerous. Traditional chemotherapy, as the mainstay of pharmacological treatment for brain tumors, suffers from several drawbacks, including poor selectivity for tumor tissues, high toxicity, a narrow therapeutic window, and a high propensity to develop multidrug resistance. Moreover, the amount of chemotherapeutic drugs that can cross the blood-brain barrier (BBB) is significantly limited. To overcome the limitations of conventional treatments, active targeting has emerged in recent years as an important strategy for enhancing the targeted delivery efficiency to brain tumor tissues.

In addition to tumor cells, the brain tumor tissues also contain tumor stem cells, vasculogenic mimicry, cerebral capillaries and the BBB, tumor neovasculature, and the blood-tumor barrier (BTB). In the early stages of the brain tumor development, the BBB remains intact and effectively restricts drug entry into the brain, which prevents approximately 98% of small-molecule chemotherapeutic drug and nearly 100% of large-molecule drugs such as proteins from crossing the BBB and reaching the brain, thereby rendering drug therapy virtually ineffective. As the tumor progresses, the BBB becomes partially disrupted and tumor neovasculature forms. However, compared to peripheral tumors, tumor neovasculature in the brain are relatively dense and have poor permeability, forming a BTB that poses a major obstacle to drug delivery. Meanwhile, the BBB persists at the invasive margins of gliomas, further impeding drug transport. Moreover, brain tumor stem cells exhibit characteristics of self-renewal, proliferation, and high tumorigenicity. Although their numbers in tumor tissue are extremely limited, they display remarkable resistance to drug treatment, easily leading to the glioma recurrence. Therefore, it is critical to develop a whole-process targeting molecule capable of crossing both the BBB and BTB, which have high affinity for both the brain tumor cell and the tumor stem cell for active targeting strategies against the brain tumors.

Glucose-regulated protein GRP78, also known as immunoglobulin heavy-chain-binding protein (Bip), is one of the major molecular chaperones in the endoplasmic reticulum and plays an essential role in protein folding and the endoplasmic reticulum stress response. In many tumor cell lines, solid tumors, and biopsy samples from human tumor tissues, the expression of GRP78 protein is significantly elevated. ^{L}VAP (L-type amino acid sequence SNTRVAP, SEQ ID NO: 1) and its stabilized and optimized peptide ^{D}VAP (D-type amino acid sequence ^{D}P^{D}A^{D}V^{D}R^{D}T^{D}N^{D}S, SEQ ID NO: 3), as well as the retro peptide of ^{L}VAP (L-type amino acid sequence PAVRTNS, SEQ ID NO: 2) and its stabilized and optimized peptide ^{S}VAP (D-type amino acid sequence ^{D}S^{D}N^{D}T^{D}R^{D}V^{D}A^{D}P, SEQ ID NO: 4), are 7-mer peptides previously validated by research team as having high binding affinity to glucose-regulated protein GRP78. These peptides can specifically target endothelial cells of tumor neovasculature and cross the BTB, as well as target tumor cells and tumor stem cells, leading to excellent targeting ability *in vivo*, but cannot cross the BBB. Moreover, recent studies have shown that many clinically used anticancer drugs such as doxorubicin (DOX), temozolomide (TMZ), 5-fluorouracil, paclitaxel, camptothecin, and bortezomib can induce endoplasmic reticulum stress and increase GRP78 protein expression.

p-hydroxybenzoic acid (pHA), a benzamide derivative, is also a compound that the research team previously verified as capable of targeting brain capillary endothelial cells (BCECs) and penetrate the BBB. Furthermore, the inventors of this invention have further covalently linked pHA with VAP, and preliminary results indicate that this conjugate exhibits the ability to target BCECs (across the BBB), cells of tumor neovasculature (across the BTB), vasculogenic mimicry, tumor cells, and tumor stem cells.

### Content of the present invention

To address the challenge of covalently linking two target molecules to achieve multifunctional targeted delivery, the present invention provides a whole-process targeting peptide, a drug complex thereof, a drug delivery system thereof, and the use thereof. Bridging structure tends to play a critically important role in realizing multifunctional targeting. Given that the original pHA-AHX-VAP peptide, constructed using aminohexanoic acid as the bridging structure, showed poor solubility, the present invention has designed a new bridging structure based on amino-monoethylene glycol-carboxylic acid or derivatives thereof (AO HX) to covalently link pHA and VAP (pHA-AOₙHX-VAP), aiming to improve the solubility of the peptide. Unexpectedly, it was found that application of AOₙHX as bridging structure significantly enhanced the peptide's whole-process targeting ability to word BCECs (across the BBB), cells of tumor neovasculature (across the BTB), vasculogenic mimicry, tumor cells, and tumor stem cells simultaneously. Moreover, the optimized peptide exhibited superior affinity to tumor cells, and the resulting drug complex exhibited improved antitumor efficacy, enhanced *in vivo* safety and other beneficial effects. Furthermore, when combined with chemotherapeutic drug, the drug complex or modified drug delivery systems based on this whole-process targeting peptide display synergistic effects, thereby further enhancing the antitumor efficacy of the chemotherapeutic drug.

To solve the above-mentioned technical problem, the first aspect of the present invention provides a whole-process targeting peptide, comprising pHA and VAP covalently linked via a bridging structure; and the VAP is ^{L}VAP, the retro peptide of ^{L}VAP, ^{D}VAP, or SVAP;
wherein, the amino acid sequence of ^{L}VAP is shown as SEQ ID NO: 1; ^{S}VAP is the D-configured peptide of ^{L}VAP; ^{D}VAP is the D-configured peptide of a retro peptide of^{L}VAP;
the bridging structure is an amino-monoethylene glycol-carboxylic acid or derivative thereof (AOₙHX), with the following general formula: 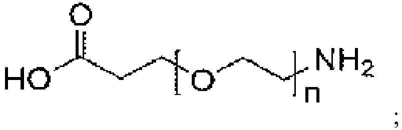
wherein, n is a natural number.

In some preferred embodiments, n is 1-20, more preferably 1-5, for example 1, 2, 3, or 5.

In some embodiments, the VAP is ^{D}VAP, and n is 1, 2, 3, or 5.

In some embodiments, the whole-process targeting peptide is pHA-AOₙHX-^{D}VAP.

In the present invention, amino-monoethylene glycol-carboxylic acid or derivatives thereof, taken as a whole, are represented by the structural formula AOₙHX shown above.

To solve the above-mentioned technical problem, the second aspect provides a targeted diagnostic peptide complex formed by linking the whole-process targeting peptide of the first aspect to an imaging moiety.

In some embodiments, the imaging moiety is an optical imaging moiety, a magnetic resonance imaging agent, and/or a radionuclide imaging agent.

In some preferred embodiments, the optical imaging moiety is selected from the following group:
(1) one or more of the fluorescent probe molecules: FITC, FAM, 6-TET, 5-TAMRA, HEX, and 6-JOE;
(2) one or more of the near-infrared dye molecules: Cy3, Cy3.5, Cy5, Cy5.5, Cy7, IR783, IR820, DiR, DiD, BODIPY630/650-X, BODIPY650/665-X, BODIPY665/676, TO-PRO-3, and TO-PRO-5;
(3) one or more of the chemiluminescent substances: luminol, isoluminol, AMPPD, CSPD, CDP-star, luciferin, and Raman probe;

the magnetic resonance imaging agent is a chelate of a Gd-based magnetic resonance substance;
and the radionuclide imaging agent is selected from one or more of the chelates of the following imaging radionuclides: ¹⁸F, ³²P, ³⁵S, ⁶⁴Cu, ^{67/68}Ga, ⁷⁵Se, ⁸⁹Zr, ⁸⁶Y, ^{99m}Tc, ^{111/111m}In, ^{123/125}I, ¹⁷⁷Lu and ^{149/161}Tb.

In some more preferred embodiments, the magnetic resonance imaging agent or radionuclide imaging agent comprises a bifunctional chelator, and a nuclide for magnetic resonance imaging or a radiopharmaceutical nuclide for imaging; wherein the bifunctional chelator is selected from one or more of the following: DOTA, DOTAGA, NOTA, NOTAGA, NODA, DTPA, TETA, CB-TE2A, Cyclam, DFO, MAG3, EC, EDTA, DADT, HYNIC, CE-DTS, and NS3.

To solve the above-mentioned technical problem, the third aspect of the present invention provides a targeted therapeutic peptide complex formed by linking the whole-process targeting peptide of the first aspect to a therapeutic drug.

In some preferred embodiments, the whole-process targeting peptide is pHA-AOₙHX-VAP; and/or, the whole-process targeting peptide is linked or condensed to the therapeutic drug directly or via a pH-sensitive hydrazone bond, or via a pH-sensitive boronate ester bond, or via a disulfide bond.

In some embodiments, the therapeutic drug is selected from one or more of the following: a tumor chemotherapeutic drug, an anti-tumor stem cell drug, a molecular-targeted drug, a peptide drug, an antibody drug, and therapeutical radionuclide chelate.

In some preferred embodiments, the tumor chemotherapeutic drug is selected from the following group:
one or more of anthracyclines, such as DOX and epirubicin;
(2) one or more of taxanes, such as paclitaxel, docetaxel, and cabazitaxel;
(3) one or more of camptothecins, such as camptothecin, hydroxycamptothecin, 9-nitrocamptothecin, and irinotecan;
(4) one or more of vinca alkaloids, such as vincristine and vinblastine;
(5) one or more of the proteasome inhibitors, such as bortezomib and carfilzomib;
the anti-tumor stem cell drug is selected from lactone-based anti-tumor drugs, such as parthenolide and derivatives thereof;
the molecular-targeted drug is selected from one or more of the following: trametinib, imatinib, nilotinib, dasatinib, everolimus, erlotinib, sunitinib, sorafenib, ibrutinib, regorafenib, vemurafenib, and olaparib;
the peptide drug is p53-activating peptide;
and the antibody-based drug is selected from one or more of the following: rituximab, bevacizumab, trastuzumab, cetuximab, pertuzumab, ipilimumab, nivolumab, and PD-L1 antibodies, as well as their genetically engineered antibody fragments, including Fab fragments, single-domain antibodies, Fv fragments, single-chain antibodies, bispecific small-molecule antibodies, microantibodies, and nanobodies;

The therapeutic radionuclide chelateis selected from one or more of the chelates of the following therapeutic radionuclides of ⁹⁰Y, ¹³¹I, ^{152/155}Tb, ¹⁵³Sm, ¹⁷⁷Lu, ^{186/188}Re, ²¹¹At, ^{212/213}Bi, ²¹²Pb, ²²⁵Ac, and ²²⁷Th.

In some more preferred embodiments, the therapeutic radionuclide chelate comprises a bifunctional chelator and a therapeutic radionuclide, wherein the bifunctional chelator is selected from one or more of the following: DOTA, DOTAGA, NOTA, NOTAGA, NODA, DTPA, TETA, CB-TE2A, Cyclam, DFO, MAG3, EC, EDTA, DADT, HYNIC, CE-DTS, and NS3.

In some more preferred embodiments, the targeted therapeutic peptide complex is selected from:
(1) pHA-AOₙHX-VAP-DOX, wherein the whole-process targeting peptide is preferably linked to DOX via a pH-sensitive hydrazone bond;
(2) pHA-AOₙHX-VAP-Paclitaxel, wherein the whole-process targeting peptide is preferably linked to paclitaxel via a disulfide bond;
(3) pHA-AOₙHX-VAP-Bortezomib, wherein the whole-process targeting peptide is preferably linked to bortezomib via a pH-sensitive boronate ester bond;
(4) pHA-AOₙHX-VAP-PMI, wherein the whole-process targeting peptide is preferably linked to the PMI via a peptide bond;
(5) pHA-AOₙHX-VAP-αPDL1, wherein the whole-process targeting peptide is preferably directly linked to αPDL1 via a thiol-maleimide addition;
(6) pHA-AOₙHX-VAP-DOTA-Gd, wherein the whole-process targeting peptide is preferably linked to Gd via the chelating agent DOTA.

In some preferred embodiments, the pHA-AOₙHX-VAP-DOX is prepared by the following steps:
the whole-process targeting peptide is stirred and mixed with tris(2-carboxyethyl)phosphine (TECP); the molar ratio of the whole-process targeting peptide to TECP is preferably 1:(4-12), for example, 1:10; and,
(2) the DOX 6-maleimidocaproyl hydrazide derivative is added, and the reaction is carried out in the dark to prepare the targeted therapeutic peptide complex; the molar ratio of the whole-process targeting peptide to DOX is 1:(2-6), for example, 1:4.

To solve the above-mentioned technical problem, the fourth aspect provides a targeted peptide-polymer carrier material formed by linking the whole-process targeting peptide of the first aspect to a polyethylene glycol-complex; wherein the complex is a lipophilic material moiety or a hydrophilic ligand moiety.

In some preferred embodiments, the lipophilic material moiety is selected from one or more of the following: phospholipids, polylactic acid, poly(lactide-co-glycolide), and polycaprolactone; and the hydrophilic ligand moiety is biotin.

To solve the above-mentioned technical problem, the fifth aspect provides a targeted drug delivery system comprising the targeted peptide-polymer carrier material of the fourth aspect and excipients; when the polyethylene glycol-complex is polyethylene glycol-phospholipid, the targeted drug delivery system can be a liposome delivery system, a micell delivery system, or a disc-shaped delivery system; when the polyethylene glycol-complex is polyethylene glycol-polylactic acid, polyethylene glycol-poly(lactide-co-glycolide), or polyethylene glycol-polycaprolactone, the targeted drug delivery system is a micell delivery system or a nanoparticle delivery system; when the polyethylene glycol-complex is polyethylene glycol-biotin, the targeted drug delivery system is a biomembrane-coated nano delivery system.

In some preferred embodiments, the excipient comprises one or more of cholesterol, phospholipids, polyethylene glycol-phospholipids, polyethylene glycol-polylactic acid, polyethylene glycol-poly(lactide-co-glycolide), and polyethylene glycol-polycaprolactone.

In some embodiments, the targeted drug delivery system is loaded with a drug, and the drug is a diagnostic agent or a therapeutic drug.

In some preferred embodiments, the diagnostic agent is the imaging moiety as defined in the targeted diagnostic peptide complex of the second aspect; and the therapeutic drug is the therapeutic drug as defined in the targeted therapeutic peptide complex of the third aspect.

To solve the above-mentioned technical problem, the sixth aspect provides a drug combination for combined therapy, comprising a chemotherapeutic drug, and a targeted therapeutic peptide complex of the third aspect or a targeted drug delivery system of the fifth aspect.

In some preferred embodiments, the chemotherapeutic drug is selected from one or more of the following: DOX, epirubicin, TMZ, paclitaxel, docetaxel, cabazitaxel, camptothecin, hydroxycamptothecin, 9-nitrocamptothecin, irinotecan, vinblastine, bortezomib, carfilzomib, and 5-fluorouracil.

In some more preferred embodiments, the administration regimen for the drug combination involves first administering a chemotherapeutic drug, followed by administering the targeted therapeutic peptide complex of the third aspect or the targeted drug delivery system of the fifth aspect after a certain period of time; wherein the certain period of time is preferably 24-48 hours.

In some specific embodiments, the chemotherapeutic drug is TMZ, and/or the targeted therapy peptide complex is pHA-AOₙHX-VAP-DOX; preferably, n is 1 or 2, and the mass ratio of TMZ to DOX in the targeted therapeutic peptide complex is 10:(1-5), for example, 10:3.

To solve the above-mentioned technical problem, the seventh aspect provides a use of the whole-process targeting peptide of the first aspect, the targeted diagnostic peptide complex of the second aspect, the targeted therapeutic peptide complex of the third aspect, the targeted peptide-polymer carrier material of the fourth aspect, the targeted drug delivery system of the fifth aspect, and/or the drug combination of the sixth aspect, in the preparation of imaging agent, tracer agent, and/or targeted therapeutic drug for a tumor such as a brain tumor or a peripheral tumor.

In this invention, the peripheral tumor refers to tumors other than those in the brain, such as a lung tumor and a breast tumor.

To solve the above-mentioned technical problem, the eighth aspect provides a whole-process targeting peptide of the first aspect, the targeted diagnostic peptide complex of the second aspect, the targeted therapeutic peptide complex of the third aspect, the targeted peptide-polymer carrier material of the fourth aspect, the targeted drug delivery system of the fifth aspect, and/or the drug combination of the sixth aspect, for use in the diagnosis and/or treatment of a tumor.

In some preferred embodiments, the tumor is a brain tumor or a peripheral tumor.

To solve the above-mentioned technical problem, the ninth aspect provides a method for diagnosing and/or treating a tumor, wherein, the method comprising administering to a subject in need a diagnostically effective dose or a therapeutically effective dose of the targeted therapeutic peptide complex of the third aspect, the targeted drug delivery system of the fifth aspect, or the drug combination of the sixth aspect.

In some preferred embodiments, the tumor is a brain tumor or a peripheral tumor.

Specifically, the invention includes:

### 1. Preparation of whole-process targeting peptide and their fluorescent labels

The whole-process targeting peptide, comprising pHA and VAP covalently linked via amino-monoethylene glycol-carboxylic acid or derivatives thereof, was formed by using a solid-phase synthesis approach; and the whole-process targeting peptide-fluorescein complex was synthesized via a Michael addition between the maleimide group and the thiol group; wherein, the structure was characterized by HPLC and MS.

### 2. In vitro targeting ability evaluation of whole-process targeting peptide

The *in vitro* affinity of the fluorescein labeled whole-process targeting peptide which was formed by covalently linking pHA and VAP via an amino-monoethylene glycol-carboxylic acid linker or derivatives thereof, to BCECs, human umbilical vein endothelial cells (HUVEC), and model tumor cells (e.g., glioma cells U87) was examined. peptide. The results were compared with that of fluorescein labeled targeting peptide formed by covalently linking pHA and VAP via aminohexanoic acid.

### 3. Preparation of the drug complex of whole-process targeting peptide

After introduction of cysteine, the whole-process targeting peptide, formed by covalently linking pHA and VAP via amino-monoethylene glycol-carboxylic acid or derivatives thereof, was reacted with a maleimide caproic hydrazide derivative on a drug, forming a peptide-drug complex containing a pH-sensitive hydrazone bond. The drugs involved in this process included anthracyclines which containing ketone or aldehyde groups, such as DOX and epirubicin.

After introduction of cysteine, the whole-process targeting peptide, formed by covalently linking pHA and VAP via amino-monoethylene glycol-carboxylic acid or derivatives thereof, was reacted with a 3-(2-pyridinedithio)propionic acid derivative, forming a peptide-drug complex containing disulfide bonds. The drugs involved in this process included taxol, docetaxel, cabazitaxel, camptothecin, hydroxycamptothecin, 9-nitrocamptothecin, irinotecan, vinblastine, vinorelbine, and other hydroxy-or amino-containing drugs.

The whole-process targeting peptide, formed by covalently linking pHA and VAP via amino-monoethylene glycol-carboxylic acid or derivatives thereof, was reacted with the boronic acid groups on the drug by modifying with dopamine, and then to form a peptide-drug complex containing pH-sensitive boronic acid esters. The drugs involved included boronate-containing compounds such as bortezomib.

The whole-process targeting peptide, formed by covalently linking pHA and VAP via amino-monoethylene glycol-carboxylic acid or derivatives thereof, was condensed with peptide drugs directly through solid-phase synthesis. The drugs involved included peptide drugs such as p53-activating peptides, antimicrobial peptides, and peptide toxins.

The whole-process targeting peptide, formed by covalently linking pHA and VAP via amino-monoethylene glycol-carboxylic acid or derivatives thereof, was conjugated to an antibody by random-site modification (activating free amino groups in the antibody and then covalently linking them to the whole-process targeting peptide) or site-specific modification (non-covalently coupling the whole-process targeting peptide to the antibody via affinity-based conjugation) to obtain an antibody complex modified with the whole-process targeting peptide. The drugs involved included rituximab, bevacizumab, trastuzumab, cetuximab, pertuzumab, ipilimumab, nivolumab, and PD-L1 antibodies, as well as antibody fragments engineered by genetic engineering techniques including Fab fragments, single-domain antibodies, Fv fragments, single-chain antibodies, bispecific small-molecule antibodies, microantibodies, and nanobodies.

After introduction of cysteine, the whole-process targeting peptide, formed by covalently linking pHA and VAP via amino-monoethylene glycol-carboxylic acid or derivatives thereof, was reacted with a maleimide group on the bifunctional chelator to obtain a whole-process targeting peptide modified radionuclide chelate peptide. The therapeutic radionuclide chelates involved include ⁹⁰Y, ¹³¹I, ^{152/155}Tb, ¹⁵³Sm, ¹⁷⁷Lu, ^{186/188}Re, ²¹¹At, ^{212/213}Bi, ²¹²Pb, ²²⁵Ac, and ²²⁷Th.

### 4. Determination of the solubility of whole-process targeting peptide-DOX complex

The solubility of a whole-process targeting peptide-DOX complex wherein pHA and VAP were covalently linked via an amino-monoethylene glycol-carboxylic acid or derivatives thereof in PBS was examined. The result was compared with that of a whole-process targeting peptide-DOX complex wherein pHA and VAP were covalently linked via aminohexanoic acid.

### 5. Evaluation of in vitro cellular uptake of whole-process targeting peptide-DOX complex.

The uptake of a whole-process targeting peptide-DOX complex wherein pHA and VAP were covalently linked via an amino-monoethylene glycol-carboxylic acid or derivatives thereof by U87 tumor cells, BCECs, and RAW264.7 cells was examined. The result was compared with that of a whole-process targeting peptide-DOX complex wherein pHA and VAP are covalently linked via aminohexanoic acid.

### 6. In vivo distribution of whole-process targeting peptide-drug complex in nude mice bearing an orthotopic glioma.

The accumulation in brain tumors of U87 orthotopic tumor model mice and the distribution across various organs of the whole-process targeting peptide-DOX complex wherein pHA and VAP were covalently linked via amino-monoethylene glycol-carboxylic acid or derivatives thereof were examined.

### 7. In vivo safety evaluation of whole-process targeting peptide-DOX complex

The normal mice were intravenously injected with DOX alone or the whole-process targeting peptide-DOX complex wherein pHA and VAP were covalently linked via amino-monoethylene glycol-carboxylic acid or derivatives thereof to evaluate the corresponding maximum tolerated dose (MTD).

### 8. Pharmacokinetic study of whole-process targeting peptide-DOX complex

The whole-process targeting peptide-DOX complex wherein pHA and VAP were covalently linked via amino-monoethylene glycol-carboxylic acid or derivatives thereof, was administered intravenously to mice. Blood samples were collected at various time points to determine DOX concentrations, thereby examining its pharmacokinetic behavior and *in vivo* distribution. The results were then compared with those of a whole-process targeting peptide-DOX complex wherein pHA and VAP were covalently linked via aminohexanoic acid.

### 9. In vivo efficacy study of whole-process targeting peptide-DOX complex

The whole-process targeting peptide-DOX complex wherein pHA and VAP were covalently linked via amino-monoethylene glycol-carboxylic acid or derivatives thereof, was administered via the tail vein of nude mice bearing U87 orthotopic tumors, and the *in vivo* antitumor efficacy was evaluated using median survival time as an indicator. The result was then compared with that of a targeted peptide-DOX complex wherein pHA and VAP were covalently linked via aminohexanoic acid.

### 10. Dose- and time-dependent upregulation of tumor GRP78 protein expression by chemotherapeutic drug

The expression of GRP78 protein in tumor cells or at the tumor site was examined using techniques such as Western blot and immunofluorescence after administering different doses of DOX, TMZ, and TMZ at different time points.

### 11. The influence of targeting effect of whole-process targeting peptide-drug complex in vivo and in vitro by chemotherapy premedication peptide

The uptake of fluorescein modified with whole-process targeting peptide wherein pHA and VAP were covalently linked via amino-monoethylene glycol-carboxylic acid or derivatives thereof by U87 cells following pretreatment with TMZ was examined.

The uptake of a whole-process targeting peptide-DOX complex wherein pHA and VAP were covalently linked via amino-monoethylene glycol-carboxylic acid or derivatives thereof by U87 cells following pretreatment with TMZ was examined.

Nude mice bearing U87 orthotopic tumor was pre-administered with TMZ, and then intravenously injected with whole-process targeting peptide fluorescein, in which pHA and VAP were covalently linked via amino-monoethylene glycol-carboxylic acid or derivatives thereof. The fluorescent distribution in the brain and normal organs of the tumor-bearing nude mice was examined using *in vivo* imaging.

### 12. Evaluation of in vivo anti-brain tumor efficacy of the combination therapy of TMZ and whole-process targeting peptide-DOX complex

After pre-administration of TMZ to nude mice bearing the U87 orthotopic tumor by oral gavage, a whole-process targeting peptide-DOX complex wherein pHA and VAP were covalently linked via amino-monoethylene glycol-carboxylic acid or derivatives thereof were administered intravenously. The *in vivo* antitumor efficacy against brain tumors was evaluated by comparing the median survival time with groups receiving saline, TMZ alone, or the whole-process targeting peptide-DOX complex.

On the basis of common knowledge in the art, the preferred conditions described above can be combined arbitrarily, thereby yielding various preferred embodiments of the present invention.

The reagents and raw materials used in this invention are all commercially available.

The positive and progressive effects of the present invention are:
The resulting peptide, formed by covalently linking pHA to VAP via amino-monoethylene glycol-carboxylic acid or derivatives thereof, not only improves the solubility of the whole-process targeting peptide, but also unexpectedly enhances its whole-process targeting ability, including crossing the BBB, targeting tumor neovasculature and crossing the blood-tumor barrier, as well as targeting vasculogenic mimicry and tumor cells, thereby having better affinity to tumor cells. Moreover, the constructed peptide-drug complex exhibits unexpected improvements in antitumor efficacy and *in vivo* safety. In addition, when used in combination with chemotherapeutic drug, the peptide-drug complex or the drug delivery system modified with the peptide shows a synergistic effect, thereby remarkably improving the anti-tumor effect.

### Brief description of the drawings

Figure 1 shows the HPLC chromatograms of pHA-AOₙHX-VAP-Cys (A and C-E in Figure 1) and the ESI-MS spectra of pHA-AO₁HX-VAP-DOX (B in Figure 1).
Figure 2 shows the HPLC and ESI-MS spectra of pHA-AO₁HX-VAP-FITC.
Figure 3 shows the uptake of fluorescein-labeled pHA-VAP with different bridging structures by primary BCECs. The figure presents quantitative (A in Figure 3) and qualitative (B in Figure 3) results from flow cytometry fluorescence analysis after BCECs were incubated for 4 hours with fluorescein-labeled pHA-AHX-VAP and pHA-AO₁HX-VAP.
Figure 4 shows the uptake of fluorescein-labeled pHA-VAP with different bridging structures by human umbilical vein endothelial cells (HUVECs). The figure presents quantitative (A in Figure 4) and qualitative (B in Figure 4) results obtained from flow cytometric fluorescence analysis after HUVECs were incubated for 4 hours with fluorescein-labeled pHA-AHX-VAP and pHA-AO₁HX-VAP.
Figure 5 shows the uptake of fluorescein-labeled pHA-VAP with different bridging structures by U87 glioma cells. The figure presents quantitative (A in Figure 5) and qualitative (B in Figure 5) results obtained from flow cytometric fluorescence analysis after incubating U87 cells with fluorescein-labeled pHA-AHX-VAP and pHA-AO₁HX-VAP for 4 hours.
Figure 6 shows the HPLC chromatograms of pHA-AOₙHX-VAP-DOX (A and C-E in Figure 6) and the ESI-MS spectrum of pHA-AO₁HX-VAP-DOX (B in Figure 6).
Figure 7 shows the solubility determination of a pHA-VAP-DOX complex with different bridge structures in PBS.
Figure 8 shows the uptake of pHA-AO₁HX-VAP-DOX by U87 cells.
Figure 9 shows the uptake of pHA-AO₁HX-VAP-DOX by RAW264.7 macrophages.
Figure 10 shows the maximum tolerated dose (MTD) study. A in Figure 10 depicts the body weight change curves of mice in the DOX group at different doses, while B in Figure 10 shows the body weight change curves of mice in the pHA-AO₁HX-VAP-DOX group at different doses.
Figure 11 shows the *in vivo* antitumor efficacy of pHA-AO₁HX-VAP-DOX against U87 orthotopic glioma; the figure depicts the survival curves of nude mice bearing the U87 orthotopic glioma.
Figure 12 shows the *in vivo* effects of pHA-AO₁HX-VAP-DOX on apoptosis and inhibition of neovascularization of U87 orthotopic glioma model mice. The figure includes TUNEL staining images of apoptotic tumor cells at the U87 orthotopic tumor site and CD31 staining images of neovascularization (A in Figure 12), as well as semi-quantitative results (B in Figure 12). White arrows point to the tumor region.
Figure 13 shows the HPLC and ESI-MS spectra of pHA-AO₁HX-VAP-Cy5.
Figure 14 shows the expression of GRP78 protein on the cell membrane of U87 cells after treatment with different concentrations of DOX. The figure presents Western blot images and semi-quantitative results of GRP78 protein expression on the cell membrane of U87 cells after incubation with different concentrations of DOX for 48 hours.
Figure 15 shows the expression of GRP78 protein on the cell membrane of U87 cells after treatment with different concentrations of pHA-AO₁HX-VAP-DOX. The figure presents Western blot images and semi-quantitative results of GRP78 protein expression on the cell membrane of U87 cells after incubation with different concentrations of pHA-AO₁HX-VAP-DOX for 48 hours.
Figure 16 shows the expression of GRP78 protein on the cell membrane of U87 cells after treatment with different concentrations of TMZ. The figure presents Western blot images and semi-quantitative results of GRP78 protein expression on the cell membrane of U87 cells after incubation with different concentrations of TMZ for 48 hours.
Figure 17 shows the expression levels of GRP78 protein on the cell membrane of U87 cells after incubation with TMZ for different durations. The figure presents Western blot images and semi-quantitative results illustrating the expression of GRP78 protein on the cell membrane of U87 cells following treatment with the same concentration of TMZ (5 µg/mL) for varying time periods.
Figure 18 shows the expression levels of GRP78 protein in tumors at different time points following TMZ treatment in nude mice bearing an orthotopic glioma; the figure depicts Western blot images and semi-quantitative result of GRP78 protein expression in nude mice bearing an orthotopic glioma at different time points following intragastric administration of TMZ (10 mg/mL).
Figure 19 shows the expression of GRP78 protein in tumors from nude mice bearing an orthotopic glioma after treatment with different concentrations of TMZ. The figure depicts immunofluorescence staining results for GRP78 protein expression in tumors from nude mice bearing an orthotopic glioma 48 hours after oral administration of different concentrations of TMZ (bar = 200 µm).
Figure 20 shows the expression levels of GRP78 protein in tumors from nude mice bearing an orthotopic glioma at different time points following TMZ administration. The figure depicts immunofluorescence staining results showing GRP78 protein expression in tumor tissues at various time points after oral administration of 10 mg/kg TMZ to nude mice bearing orthotopic gliomas (bar = 200 µm).
Figure 21 shows the expression levels of GRP78 protein in normal brain tissue at different time points following TMZ administration to nude mice bearing an orthotopic glioma. The figure depicts immunofluorescence staining results showing GRP78 protein expression in normal brain tissue at various time points after oral administration of 10 mg/kg TMZ to nude mice bearing an orthotopic glioma (bar = 200 µm).
Figure 22 shows the uptake of pHA-AO₁HX-VAP-fluorescein by U87 cells following pretreatment with TMZ. The figure depicts the uptake of fluorescein modified by pHA-AO₁HX-VAP in U87 cells after treatment with 5 µg/mL TMZ for 48 hours.
Figure 23 shows the uptake of pHA-AO₁HX-VAP-DOX by U87 cells following pretreatment with TMZ. The figure depicts the uptake of the whole-process targeting peptide-drug complex (pHA-AO₁HX-VAP-DOX) by U87 cells after treatment with 5 µg/mL TMZ for 48 hours.
Figure 24 shows the distribution of pHA-AO₁HX-VAP-Cy5 in the brains of nude mice bearing an orthotopic glioma model after pretreatment with TMZ. Specifically, A in Figure 24 illustrates the experimental design principles; B-C in Figure 24 present the time-dependent changes in fluorescent distribution within the brain as observed by *in vivo* imaging; D and E in Figure 24 and depict the distribution of the pHA-AO₁HX-VAP-modified luciferin in brain tumors and normal organs, respectively.
Figure 25 shows the therapeutic efficacy of TMZ combined with pHA-AO₁HX-VAP-DOX against orthotopic glioma. A in Figure 25 illustrates that pre-administration of TMZ in combination with pHA-AO₁HX-VAP-DOX significantly prolonged the median survival time of mice bearing an orthotopic glioma model (n = 10). B in Figure 25 depicts the body weight change curves of mice treated with TMZ pre-administration combined with pHA-AO₁HX-VAP-DOX against orthotopic glioma.
Figure 26 shows HE-stained brain sections from mice bearing intracranial gliomas, taken in situ. The figure depicts the results of HE staining of the largest cross-sections of mouse brain tumors at the end of treatment for each group (bar = 1 mm).
Figure 27 shows the uptake of pHA-AOₙHX-VAP-DOX by U87 cells.
Figure 28 shows the uptake of pHA-AO₁HX-VAP-DOX by BCECs.
Figure 29 shows the accumulation of pHA-AOₙHX-VAP-DOX in gliomas after intravenous administration to nude mice bearing an orthotopic glioma.
Figure 30 shows the distribution of pHA-AOₙHX-VAP-DOX in various organs after intravenous administration to nude mice bearing an orthotopic glioma.

### Detailed description of the preferred embodiment

The present invention is further illustrated below by the way of example, but the invention is not thereby limited to the scope of the described examples. The experimental methods for which specific conditions are not indicated in the following examples were selected according to the conventional methods and conditions, or according to the commodity specification.

In the present invention, the AOₙHX refers to the amino-monoethylene glycol-carboxylic acid or derivatives thereof, and AHX refers to the aminohexanoic acid.

### Example 1 Preparation and characterization of a whole-process targeting peptide and its fluorescent and drug complex

### 1.1 Preparation and characterization of the pHA-AOₙHX-VAP-Cys peptide

The peptide pHA-AOₙHX-VAP-Cys (sequence: pHA-amino-various ethylene glycol units- carboxylic acid-Cys- ^{D}P^{D}A^{D}V^{D}R^{D}T^{D}N^{D}S) was prepared using the Fmoc solid-phase peptide synthesis method. AOₙHX refers to amino-ethylene glycol-carboxylic acid with varying ethylene glycol units, wherein n denotes the number of ethylene glycol units.

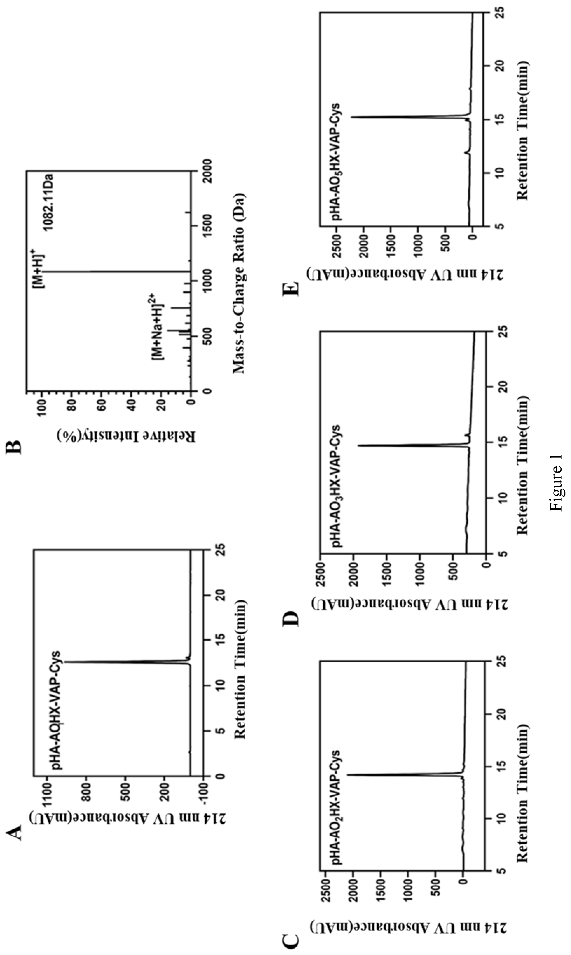

Detailed method: Using HBTU/DIEA as the condensing agent and a 20% piperidine solution in DMF as the deprotection reagent, Fmoc-D-Ser(tBu)-2-Chlorotrityl Resin was sequentially loaded with amino acids in the order from the carboxyl-terminus toward the amino-terminus. Subsequently, Fmoc-amino-AOₙHX-carboxylic acids containing different ethylene glycol units (n=1, 2, 3, and 5) and para-hydroxybenzoic acid were added one after another. After completion of the synthesis, the peptide was cleaved from the resin. The crude peptide was then purified by high-performance liquid chromatography using an acetonitrile/water system (containing 0.1% TFA) as eluent. The purified peptide was characterized by HPLC and mass spectrometry, and the results were shown in Figure 1. Chromatographic conditions used in Figure 1: column (YMC, C18): 150 × 4.6 mm; mobile phase A: Water (containing 0.1% trifluoroacetic acid); mobile phase B: Acetonitrile (containing 0.1% trifluoroacetic acid); gradient program: 2-32 min, 5% B-65% B; flow rate: 0.7 mL/min; column temperature: 40°C; detection: UV at 214 nm; retention times: 12.56 min (pHA-AO₁HX-VAP-Cys), 14.21 min (pHA-AO₂HX-VAP-Cys), 14.70 min (pHA-AO₃HX-VAP-Cys), 15.25 min (pHA-AO₅HX-VAP-Cys), as shown in A and C-E in Figure 1. As shown in B in Figure 1, the molecular weight of pHA-AO₁HX-VAP-Cys determined by ESI-MS analysis was 1082.11, which is consistent with the theoretical value.

### 1.2 Synthesis and characterization of pHA-AO₁HX-VAP-Fluorescein and pHA-AO₁HX-VAP-Cy5

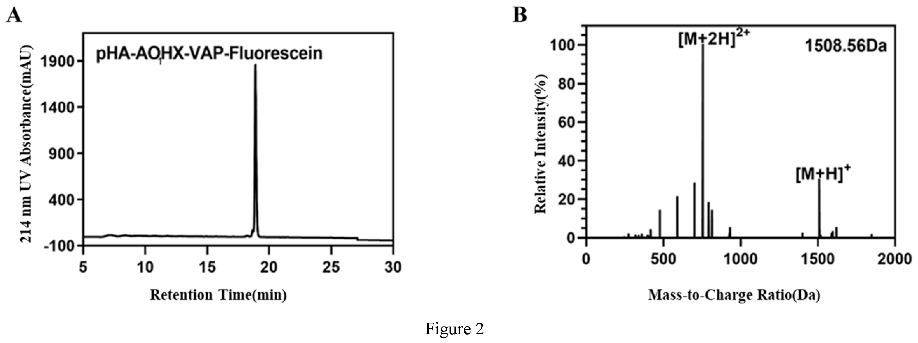

The pHA-AO₁HX-VAP-Cys obtained from the above steps was dissolved in a 0.1 M PBS solution (pH 7.2). Fluorescein-5-maleimide was dissolved in DMF, then the two solutions were mixed and stirred magnetically until the reaction was complete, as monitored by HPLC. After the reaction was entirely completed, the reaction was stopped and the solutions were proceeded with preparative liquid-phase purification, using an acetonitrile/water system (containing 0.1% TFA) for separation and purification. The mixture was freeze-dried to obtain the pure product pHA-AO₁HX-VAP-Fluorescein. The HPLC and mass spectra were shown in Figure 2. The chromatographic conditions in Figure 2 were identical to those in Figure 1with a retention time of 18.90 min. The molecular weight determined by ESI-MS was 1508.56, which is consistent with the theoretical value.

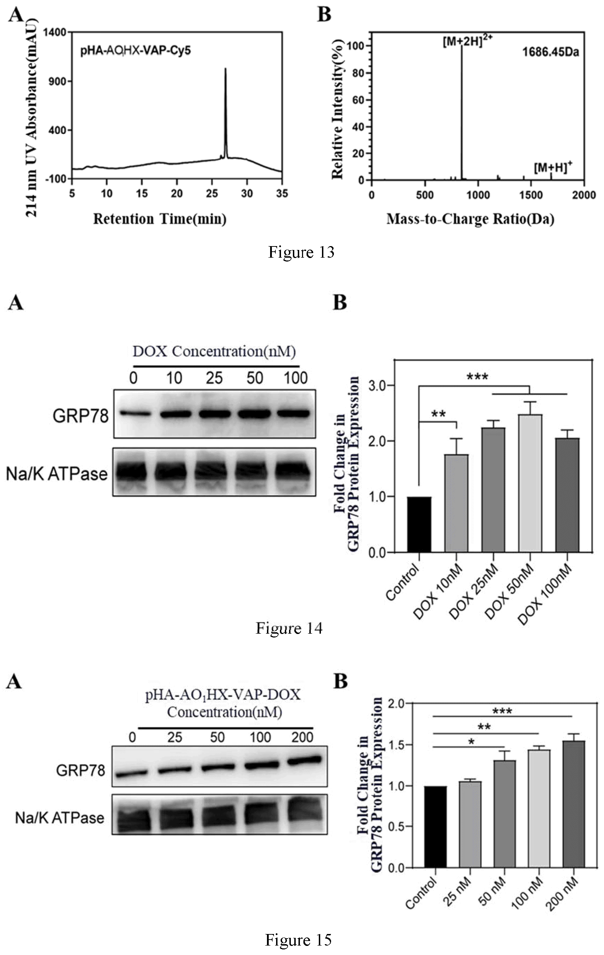

pHA-AO₁HX-VAP-Cy5 was prepared using the same method as described above; characterization results were shown in Figure 13. The chromatographic conditions used for Figure 13 were identical to those for Figure 1, with a retention time of 26.92 min. The molecular weight determined by ESI-MS was 1686.45, which is consistent with the theoretical value.

### 1.3 Preparation of the pHA-AOₙHX-VAP drug complex

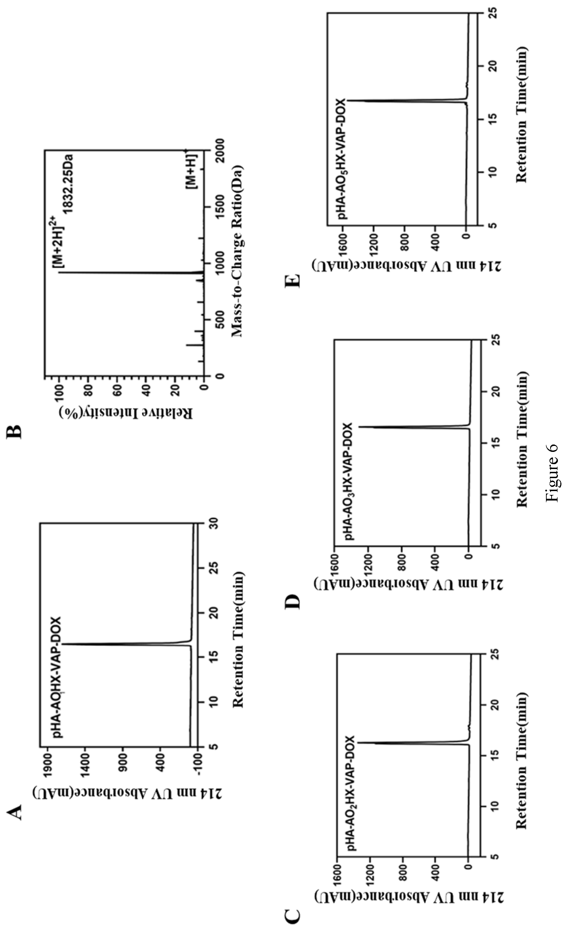

pHA-AOₙHX-VAP-DOX were prepared as examples of whole-process targeting peptide conjugated with drugs containing ketone or aldehyde groups. The pHA-AOₙHX-VAP peptide (n = 1, 2, 3, 5) was dissolved in phosphate-buffered saline (0.1 M, pH 7.4), and 10-fold molar excess of TCEP was added. The mixture was stirred at 4°C for 20 min. Subsequently, 4-fold molar excess of DOX 6-maleimidocaproyl hydrazide derivative was added, and the reaction was allowed to proceed in the dark at room temperature for 1 hour. The reaction mixture was purified by preparative liquid chromatography and freeze-dried to afford pHA-AOₙHX-VAP-DOX (n = 1, 2, 3, 5). HPLC and mass spectra are shown in Figure 6. The chromatographic conditions used are as follows: column (YMC, C18): 150 × 4.6 mm; mobile phase A: water containing 0.01% formic acid; mobile phase B: acetonitrile containing 0.01% formic acid; gradient elution program: 2-32 min, 5% B-65% B; flow rate: 0.7 mL/min; column temperature: 40°C; detection: UV at 214 nm; retention times: 16.51 min (pHA-AO₁HX-VAP-DOX), 16.26 min (pHA-AO₂HX-VAP-DOX), 16.59 min (pHA-AO₃HX-VAP-DOX), and 16.76 min (pHA-AO₅HX-VAP-DOX), as shown in A and C-E in Figure 6. As shown in B in Figure 6, ESI-MS analysis reveals that the molecular weight of pHA-AO₁HX-VAP-DOX was 1832.25 Da, which is consistent with the theoretical molecular value.

pHA-AOₙHX-VAP-paclitaxel was prepared as an example of whole-process targeting peptide linked to drugs via a disulfide bond. 200 mg of paclitaxel was dissolved in 10 mL of chloroform, cooled to 0-5°C, and sequentially 39.99 mg of DCC and 60.4 mg of 3-(2-pyridinedithio)propionic acid were added. Once the addition was complete, the reaction mixture was allowed to warm to room temperature and stirred overnight. The reaction mixture was then filtered and purified by column chromatography (eluted with CHCl₃/MeOH at a ratio of 50:1 to 15:1, v/v), thereby yielding the paclitaxel 3-(2-pyridinedithio)propionic acid derivative. The paclitaxel 3-(2-pyridinedithio)propionic acid derivative was dissolved in 5 mL of DMF. 1.5 times the molar amount of pHA-AOₙHX-VAP-Cys (n = 1, 2, 3, 5) was dissolved in PBS/DMF, and the pH of the solution was maintained between 4 and 5. The paclitaxel 3-(2-pyridinedithio)propionic acid derivative was then added dropwise to the thiol-containing peptide solution and allowed to react at room temperature for 6 hours. The resulting peptide-paclitaxel complex was subsequently purified using preparative liquid chromatography and lyophilized.

pHA-AOₙHX-VAP-bortezomib (n = 1, 2, 3, 5) was prepared as an example of whole-process targeting peptide modified drug at the N-terminus. First, amino acids were sequentially coupled onto the resin according to the peptide synthesis protocol. Once all amino acid residues of the peptide coupling completed, the Boc protecting group at the N-terminus was removed by treatment with trifluoroacetic acid. Next, a DMF solution containing 3-fold molar excess of succinic anhydride and DIEA was added, and the mixture was allowed to react at room temperature for 30 minutes. After washing the resin, 5-fold molar excess of trimethylchlorosilane protected dopamine was added, and the reaction was carried out at room temperature for 1 hour using HBTU/DIEA as the coupling agent. The resin was then cleaved with HF, and the resulting peptide-dopamine derivative was purified by preparative HPLC. In a buffer solution at pH 7.4, the peptide-dopamine derivative was mixed with bortezomib in a molar ratio of 1:1 to obtain the peptide-bortezomib complex.

AOₙHX-VAP-PMI (n = 1, 2, 3, 5) was prepared as an example of a whole-process targeting peptide-fused peptide drug. It was directly prepared via solid-phase peptide synthesis. The specific method involved first determining the sequence of the pHA-AOₙHX-VAP-PMI peptide, then sequentially incorporating amino acids according to the same procedure used for peptide preparation. After cleavage with HF and subsequent purification, the final product pHA-AOₙHX-VAP-PMI was obtained.

pHA-AOₙHX-VAP-PD-L1 (n = 1, 2, 3, 5) was prepared as an example of a whole-process targeting peptide-modified antibody drug is. In an αPDL1 solution, Sulfo-SMCC (1 mg/mL) was added and reacted at room temperature for 30 minutes. The excess Sulfo-SMCC was removed by dialysis through a desalting column. Then, the PBS solution containing pHA-AOₙHX-VAP-Cys was added, thoroughly mixed, and was incubated at 4°C for 12 hours. Subsequently, the react solution was dialyzed twice (each time for 30 minutes) in a 14 K Da dialysis bag using pure water. The liquid from the dialysis bag was collected to obtain the whole-process targeting peptide-modified PDL1 antibody complex (pHA-AOₙHX-VAP-αPDL1).

pHA-AOₙHX-VAP-DOTA-Gd (n = 1, 2, 3, 5) was prepared as an example of radionuclide chelate modified with multi-targeting peptide for diagnostic or therapeutic purposes. 0.1 mmol of pHA-AOₙHX-VAP-Cys was dissolved in PBS at pH 7, then 0.1 mmol of MAL-DOTA was added, and the mixture was stirred for 1 hour. The reaction mixture was purified by preparative liquid chromatography and freeze-dried to obtain pHA-AOₙHX-VAP-DOTA. The freeze-dried pHA-AOₙHX-VAP-DOTA was dissolved in an aqueous ammonium acetate solution, followed by the addition of GdCl₃·6H₂O. The mixture was stirred at room temperature for 1 hour and was further purified by preparative chromatography and then, was freeze-dried to yield pHA-AOₙHX-VAP-DOTA-Gd.

### Example 2 In vitro targeting validation of the whole-process targeting peptide

### 2.1 In vitro targeting of a whole-process targeting peptide toward primary brain capillary endothelial cells (BCECs)

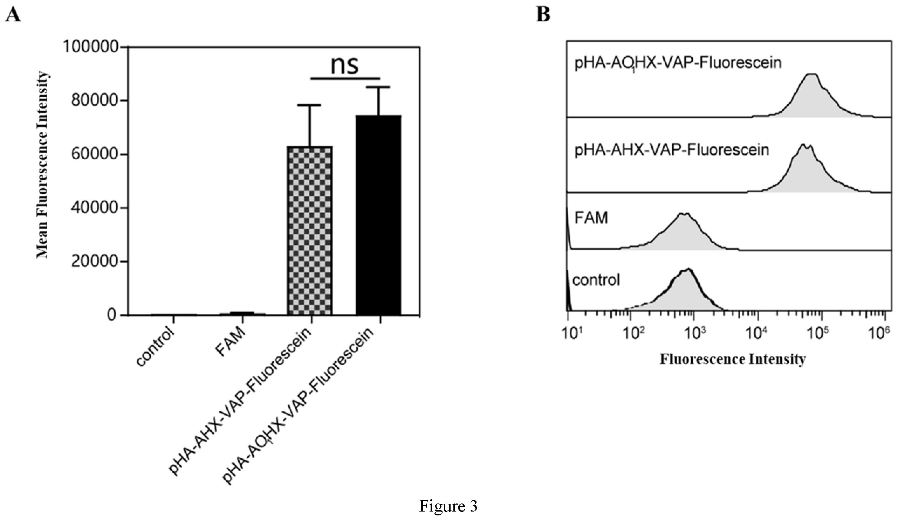

After decapitation, brains were collected from 4-week-old SD rats and rapidly isolated in pre-cooled D-Hanks solution to obtain the cerebral cortex. The meninges and major blood vessels of the brain were wholly removed, the tissue was minced, and then digested at 37°C for 90 minutes with the addition of collagenase and DNase. Following centrifugation at 1,000 rpm for 8 minutes, the supernatant was discarded. The resulting pellet was resuspended in DMEM containing 20% BSA and centrifuged at 1,000 g for 20 minutes at 4°C. The supernatant and upper layer were wholly removed, and the bottom microvessel fraction was transferred into DMEM culture medium. After another centrifugation at 1,000 rpm for 5 minutes, the microvessel fraction was resuspended in DMEM medium supplemented with 20% fetal bovine serum. The resuspended microvessels were seeded into 12-well plates and cultured at 37°C under 5% CO₂ and saturated humidity conditions for 24 hours. Subsequently, the cells were switched to endothelial-specific culture medium containing puromycin and further cultured for 72 hours. Then, the cells were switched again to endothelial-specific culture medium supplemented with cell growth factors and cultured for another 72 hours to obtain primary BCECs. A fluorescent labeled peptide solution at a concentration of 5 µM was prepared using DMEM medium supplemented with 10% FBS, and the DMEM medium in the 12-well plates was replaced with this fluorescent labeled peptide solution. The cells were incubated at 37°C for 4 hours, after which the fluorescein solution was discarded. The plate was washed twice with PBS, followed by treatment with trypsin to digest the cells. The cells were then dispersed in DMEM culture medium, centrifuged, and the supernatant was discarded. The cells were washed twice with PBS, and finally, 200 µL of PBS was added to each well to resuspend the cells. Flow cytometry was used to analyze the samples. The results were shown in Figure 3. As can be seen in the figure, there was no significant difference in the uptake of pHA-AO₁HX-VAP and pHA-AHX-VAP by BCECs.

### 2.2 In vitro targeting of the whole-process targeting peptide toward human umbilical vein endothelial cells (HUVECs)

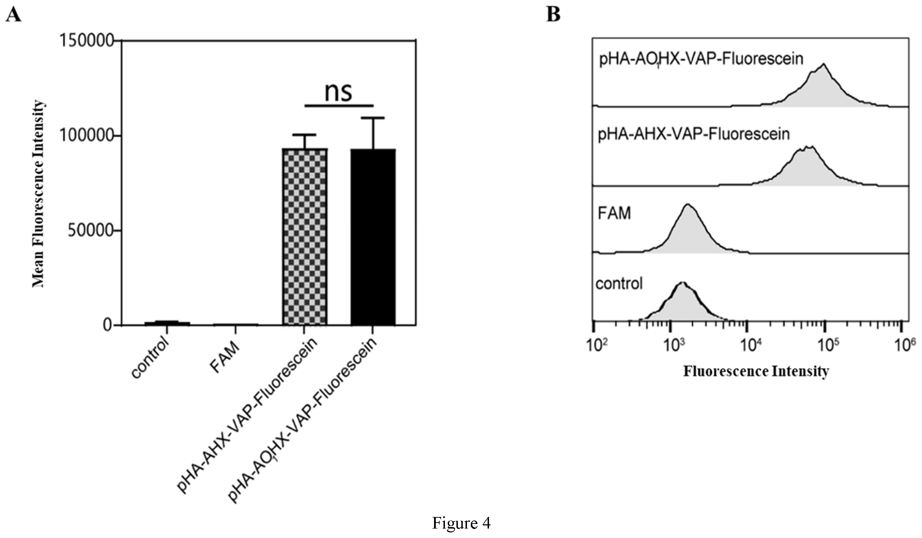

HUVEC cells grown in monolayer cultures during the logarithmic growth phase were detached from the monolayer using 0.25% trypsin. The single-cell suspension was prepared in DMEM medium supplemented with 10% fetal bovine serum. Cells were seeded into 12-well culture plates at a density of 1 × 10⁵ cells per well, with a volume of 1 mL per well. The plates were then transferred to a CO₂ incubator and cultured for 24 hours under conditions of 37°C, 5% CO₂, and saturated humidity. The experiment was carried out as described above. Flow cytometry analysis results were shown in Figure 4. As can be seen in the figure, there was no significant difference in the uptake of pHA-AO₁HX-VAP and pHA-AHX-VAP by HUVEC cells.

### 2.3 In vitro targeting of a whole-process targeting peptide toward U87 glioma cells.

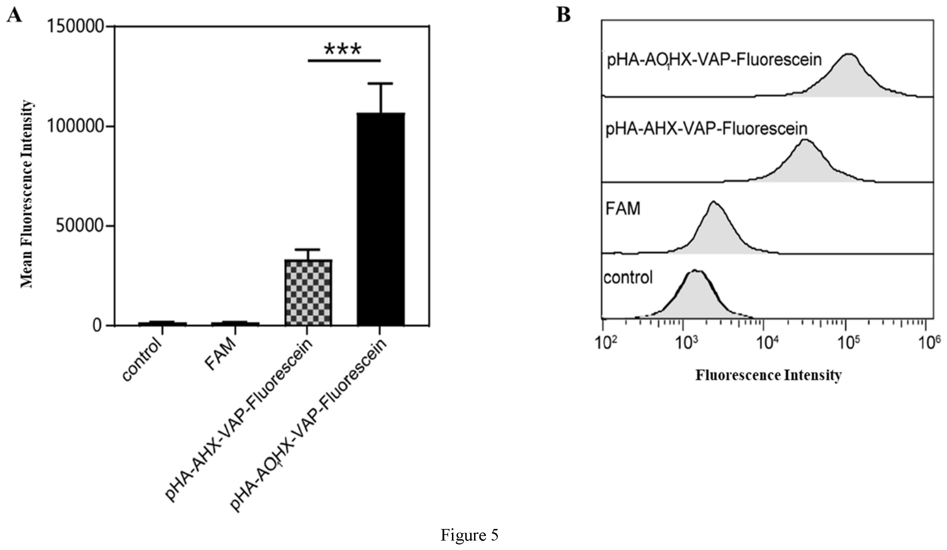

U87 cells grown in monolayer cultures during the logarithmic growth phase were tested as above. The results of flow cytometric analysis are shown in Figure 5. As can be seen in the figure, U87 cells exhibit significantly higher uptake of pHA-AO₁HX-VAP compared to pHA-AHX-VAP, suggesting that pHA-AO₁HX-VAP has better targeting ability for glioma cells.

### Example 3 Determination of the saturation solubility of the whole-process targeting peptide-drug complex

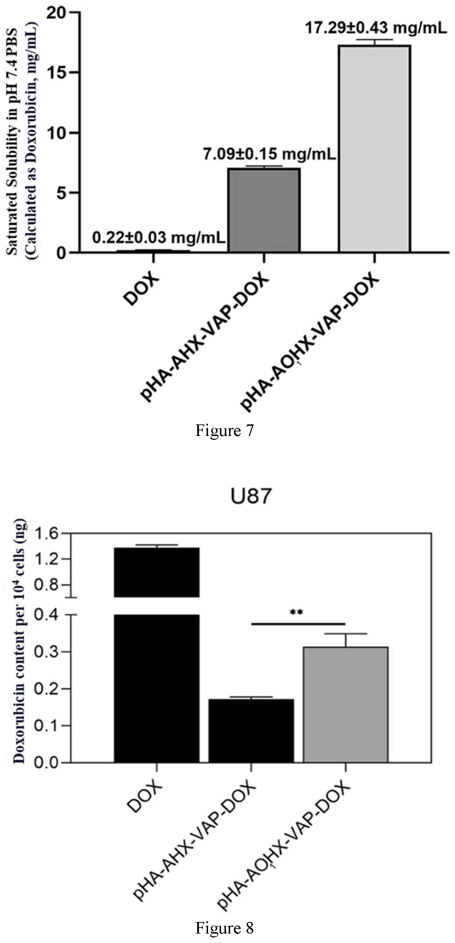

A certain amount of DOX, pHA-AHX-VAP-DOX, and pHA-AO₁HX-VAP-DOX was weighed and dispersed in 100 µL of PBS (pH 7.4). The mixture was incubated at 37°C for 24 hours, then filtered through a membrane of 0.22 µm. The filtrates were subsequently diluted 10-fold and 20-fold, respectively, and the concentration of DOX was measured. The results were shown in Figure 7. Free DOX exhibits poor water solubility; its saturation solubility in PBS buffer at pH 7.4 is 0.22 ± 0.03 mg/mL. After forming a covalent complex with the peptide, the solubility of DOX significantly increased due to the inherent hydrophilicity of the peptide. Specifically, the saturation solubility of pHA-AHX-VAP-DOX was 7.09 ± 0.15 mg/mL (calculated based on DOX), while that of pHA-AO₁HX-VAP-DOX was 17.29 ± 0.43 mg/mL. Therefore, the solubility of these complexes was 32-fold and 78-fold higher than that of free DOX, respectively. From a solubility perspective, pHA-AO₁HX-VAP-DOX is expected to have better potential for *in vivo* applications as it can avoid the use of organic solvents, allowing for higher drug doses and improved therapeutic efficacy.

### Example 4 Intracellular DOX dosage detection

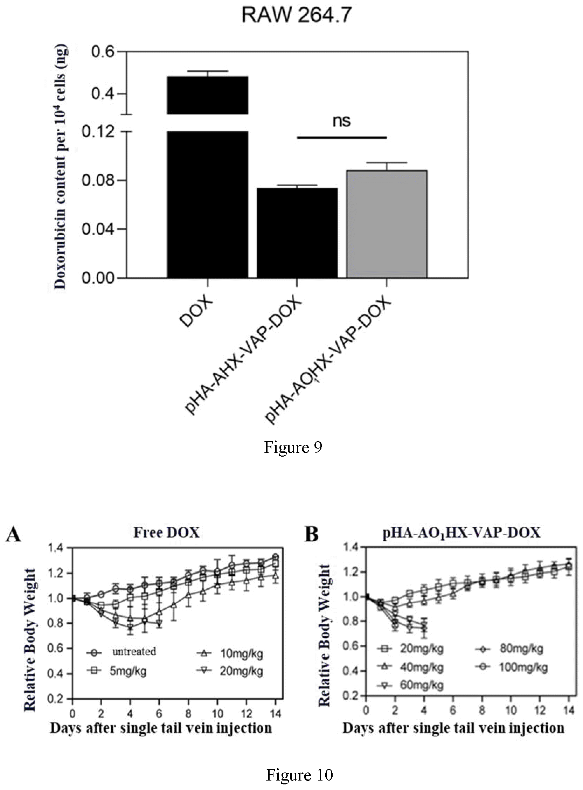

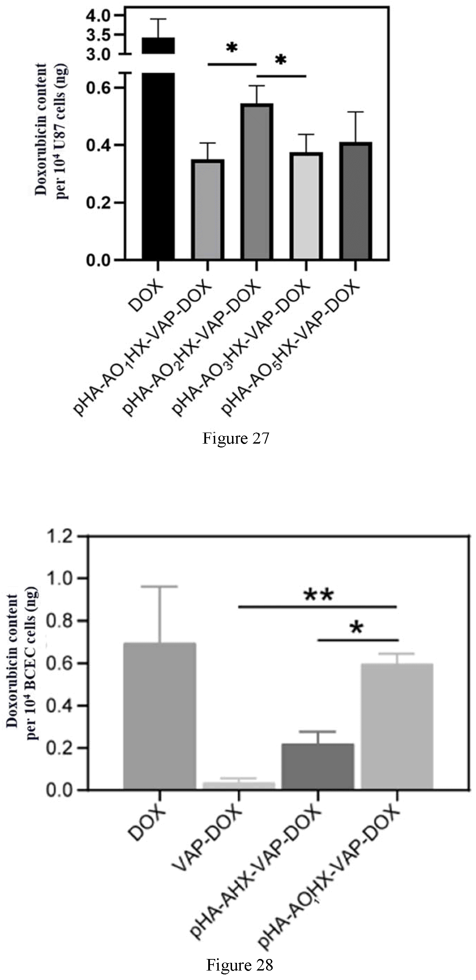

Tumor cells U87 in the logarithmic growth phase and macrophages RAW264.7, both cultured as monolayers, were detached from the culture dishes by digestion with 0.25% trypsin. The single-cell suspensions were prepared in DMEM medium supplemented with 10% fetal bovine serum. Each well of a 6-well plate was seeded with 5 × 10⁵ cells in a volume of 2 mL per well. The plates were then transferred to a CO₂ incubator and cultured overnight at 37°C under conditions of 5% CO₂ and saturated humidity. After overnight incubation, the culture medium was wholly aspirated from each well. DOX, pHA-AHX-VAP-DOX, and pHA-AOₙHX-VAP-DOX (n = 1, 2, 3, 5) solutions were added to the wells, and the cells were incubated at 37°C for 4 hours. The supernatant was then discarded. Following cell collection by trypsinization, the cells were washed three times with PBS and counted. After the final wash, any remaining supernatant was wholly removed. To the cell pellet, acidified methanol (Vₘₑₜₕₐₙₒₗ : V_{methanoic acid} = 98:2) was added, and the cells were subjected to sonication. After centrifugation at 10,000 rpm for 10 minutes, the supernatant was collected and analyzed for DOX concentration. The results were shown in Figure 8, 9, and 27. As shown in Figure 8, free DOX, due to its direct contact with cells, can passively diffuse into the cells, resulting in higher cellular uptake. Whole-process targeting peptide-DOX complex was internalized via receptor-mediated endocytosis; among which, U87 cells exhibited significantly higher uptake of pHA-AO₁HX-VAP-DOX compared to pHA-AHX-VAP-DOX. As illustrated in Figure 9, compared to the model tumor cells U87, markedly reduced uptake of the whole-process targeting peptide-DOX complex by macrophages was observed, suggesting that it exhibits excellent targeting specificity. As shown in Figure 27, pHA-AOₙHX-VAP-DOX (n = 1, 2, 3, 5) containing different ethylene glycol units can also be internalized into cells via receptor-mediated endocytosis; wherein, the best uptake of pHA-AO₂HX-VAP-DOX was demonstrated by U87 cells.

Primary BCECs were isolated from 4-week-old SD rats and seeded in 6-well plates. After one week of culture, the culture medium in the plates was aspirated, and solutions containing DOX, VAP-DOX, pHA-AHX-VAP-DOX, and pHA-AO₁HX-VAP-DOX were added separately. The plates were incubated at 37°C for 4 hours, after which the supernatant was discarded. The remaining steps were performed as described above, and the supernatant was collected to determine the concentration of DOX. As shown in Figure 28, GRP78 protein expression is absent in BCECs, leading to a relatively low intracellular DOX concentration induced by VAP-DOX. However, when modification with the whole-process targeting peptide, the drug was internalized in cells with significantly increased concentration, and the intracellular drug concentration achieved by pHA-AO₁HX-VAP-DOX was even higher than that achieved by pHA-AHX-VAP-DOX.

### Example 5 In vivo distribution of the whole-process targeting peptide -drug complex in a nude mouse model of orthotopic glioma

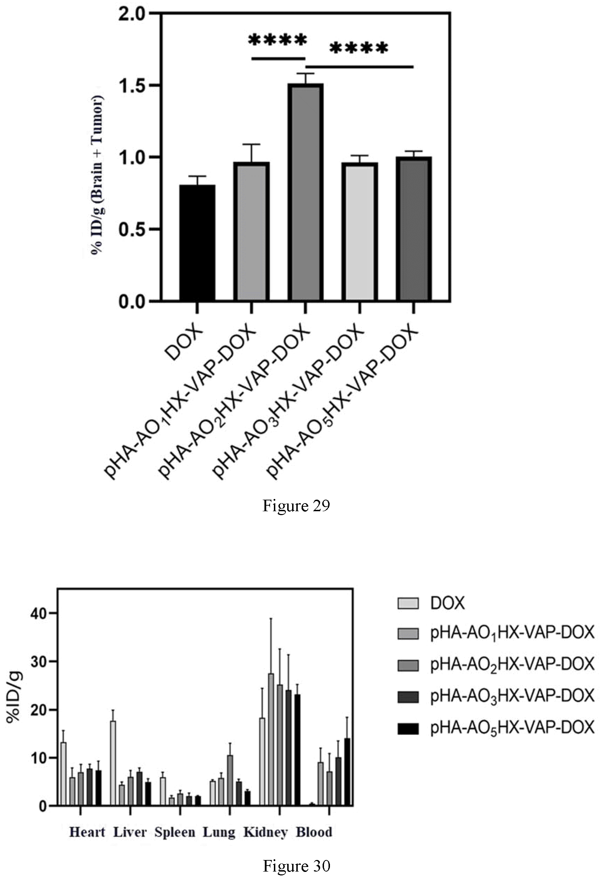

Approximately 22 g nude mice were anesthetized and fixed onto a stereotaxic apparatus. An orthotopic glioma mouse model was established by injecting 5 µL of U87 cell suspension (8×10⁵ cells) with microsyringe into the striatum of the mice (0.6 mm anteroposterior to the bregma, 1.8 mm lateral to the midline, and 3 mm ventral to the surface). Ten days after tumor implantation, the mice were administered DOX (5 mg/kg) or pHA-AOₙHX-VAP-DOX (n = 1, 2, 3, 5; DOX dose: 5 mg/kg) via tail vein injection. Thirty minutes later, the mice were anesthetized, perfused with PBS, and the major organs including the brain, heart, liver, spleen, lungs, and kidneys were collected. The tissues from each organ were blotted dry with filter paper and weighed. Then, according to the weight of each organ, a specific volume of isopropanol containing 0.075 M HCl and 10% water was added, followed by homogenization. After overnight incubation at 4°C, the samples were centrifuged, and the supernatants were collected for analysis. The results were shown in Figure 29 and 30. As shown in Figure 29, the brain accumulation of DOX was significantly increased in the groups treated with pHA-AOₙHX-VAP-DOX (n = 1, 2, 3, 5) compared to that of free DOX, with the greatest increase observed in the group treated with pHA-AO₂HX-VAP-DOX. As depicted in Figure 30, the accumulation of DOX in the heart was reduced by pHA-AOₙHX-VAP-DOX (n = 1, 2, 3, 5), while the drug concentration in the blood was increased.

### Example 6 Study of the maximum tolerated dose

Male Balb/c mice, five in each group, were administered DOX (5 mg/kg, 10 mg/kg, 20 mg/kg) via the tail vein, as well as pHA-AO₁HX-VAP-DOX at doses corresponding to DOX levels of 20 mg/kg, 40 mg/kg, 60 mg/kg, 80 mg/kg, and 100 mg/kg. The body weight changes of the mice were recorded daily (relative body weight change = daily body weight / initial body weight), with continuous monitoring for 14 days to observe whether any mice died. In Figure 10, the MTD was defined as the dose at which no mice died, whereas mice administered with pHA-AHX-VAP-DOX at the equivalent DOX dose experienced mortality. As shown in Figure 10, the MTD for free DOX was approximately 10 mg/kg, whereas the MTD for pHA-AO₁HX-VAP-DOX was approximately 40 mg/kg, indicating a significant improvement in *in vivo* safety.

### Example 7 In vivo pharmacodynamic study of the whole-process targeting peptide -drug complex

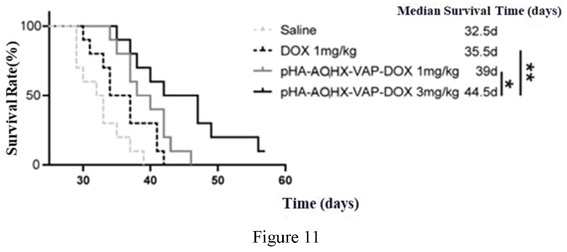

Approximately 20 g nude mice were anesthetized and fixed onto a stereotaxic apparatus. Orthotopic glioma mouse model was established by injecting 5 µL of U87 cell suspension (5×10⁵ cells) with microsyringe into the striatum of the mice (0.6 mm anteroposterior to the bregma, 1.8 mm lateral to the midline, and 3 mm deep). On day 5 after tumor implantation, the mice were randomly divided into four groups, with 10 mice per group. Every other day, the mice intravenously injected with saline, DOX (single dose of 1 mg/kg), or pHA-AO₁HX-VAP-DOX (containing single doses of DOX at 1 mg/kg and 3 mg/kg, respectively). The time of death for each group of mice was recorded, and survival curves were plotted; the results were shown in Figure 11. Using median survival time in the animal model as an indicator, compared with the saline group (median survival time: 32.5 days) and the free DOX group (median survival time: 35.5 days), a significantly prolonged median survival time was showed in the pHA-AO₁HX-VAP-DOX group (the median survival time of 1 mg/kg group and 3 mg/kg group were 39 and 44.5 days, respectively), and a dose-dependent effect was observed.

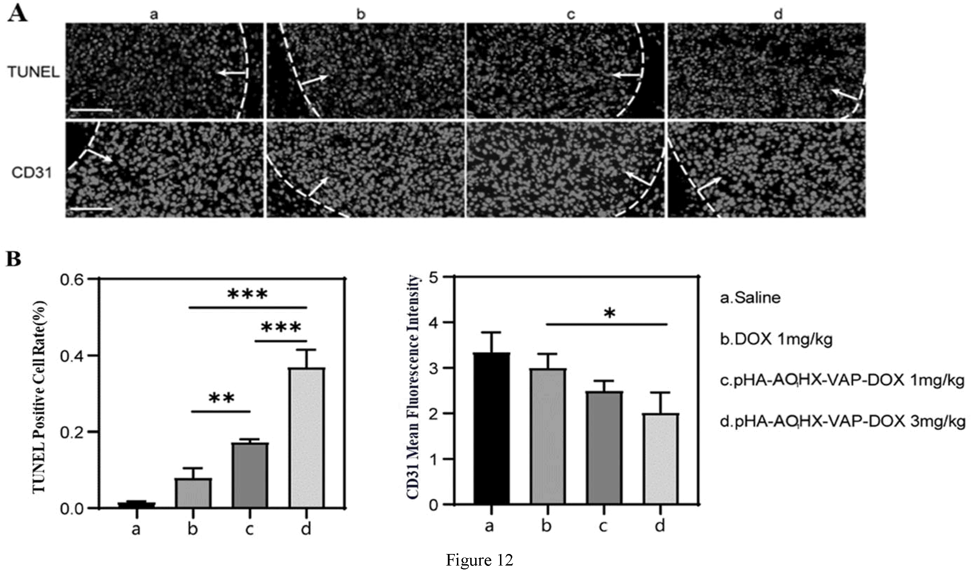

On the second day after drug administration, the tumor-bearing nude mice were euthanized, and the tumor tissues were excised for fixation. Paraffin sections or frozen sections were prepared, and the inhibition of neovascularization was assessed by CD31 staining, while the promotion of apoptosis was evaluated by TUNEL staining. The results were shown in Figure 12. As illustrated in Figure 12, compared with free DOX, DOX modified with pHA-AO₁HX-VAP significantly promoted tumor apoptosis and inhibited neovascularization.

### Example 8: Western blot detection of GRP78 protein expression

After cells were subjected to various treatments, the culture medium was removed, and the cells were washed twice with pre-cooled PBS. The cells were then scraped off and collected into an EP tube. Membrane proteins were extracted using a cell membrane protein extraction kit, followed by centrifugation at 12,000 rpm at 4°C to obtain the supernatant, which was stored for later use. The total protein concentration was determined using the BCA assay, after which the sample was mixed with 5× loading buffer at a ratio of 4:1 and heated to denature the proteins. The samples were stored at -20°C for future use. Samples containing approximately 40 µg of protein were subjected to 15% polyacrylamide gel electrophoresis and then transferred onto polyvinylidene fluoride (PVDF) membranes. The membranes were blocked at room temperature for 1 hour in TBST containing 5% nonfat dry milk, and then incubated overnight at 4°C with the corresponding primary antibody. After three washes with TBST, the membranes were incubated at room temperature for 1 hour with HRP-conjugated secondary antibodies, followed by three additional washes with TBST. According to the manufacturer's instructions, the enhanced chemiluminescence protein blotting detection kit (ECL) was used to develop and detect protein bands on a gel imaging system.

After U87 cells were treated with free DOX at different concentrations for 48 hours, membrane proteins were extracted and detected using the method described above. The results were shown in Figure 14. As illustrated in Figure 14, following treatment with DOX at various concentrations, the expression of GRP78 protein on the cell membrane surface was significantly upregulated in U87 cells.

After U87 cells were treated with different concentrations of pHA-AO₁HX-VAP-DOX (concentrations expressed in terms of DOX) for 48 hours, membrane proteins were extracted and analyzed using the method described above. The results were shown in Figure 15. As illustrated in Figure 15, following treatment with various concentrations of pHA-AO₁HX-VAP-DOX, the expression of GRP78 protein on the cell membrane surface of U87 cells was significantly upregulated.

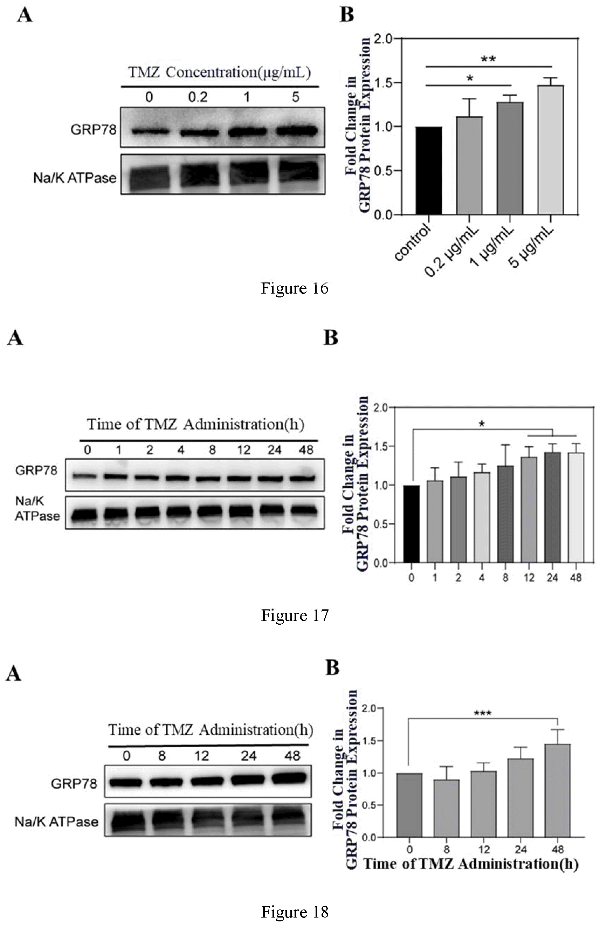

After U87 cells were treated with different concentrations of TMZ for 48 hours, membrane proteins were extracted and detected using the method described above. The results were shown in Figure 16. As illustrated in Figure 16, following treatment with different concentrations of TMZ, the expression of GRP78 protein on the cell membrane surface was significantly upregulated in U87 cells.

After U87 cells were treated with 5 µg/mL TMZ for varying durations, membrane proteins were extracted and analyzed using the method described above. The results were shown in Figure 17. As illustrated in Figure 17, following TMZ treatment, the GRP78 protein on the cell membrane began to significantly upregulate after 12 hours and continued to show a significant increase on the cell membrane surface up to 48 hours.

After administering TMZ at a concentration of 10 mg/mL by gavage to mice bearing orthotopic glioma tumors at different time points, the mice were euthanized, and brain tumors were excised. Membrane proteins were then extracted and analyzed using the method described above; the results were shown in Figure 18. As illustrated in Figure 18, following TMZ treatment in the orthotopic glioma-bearing nude mice, the expression of GRP78 protein in tumor tissue was significantly upregulated after 48 hours, reaching 1.45 times that of the control group.

### Example 9 Immunofluorescence detection of GRP78 protein expression

After various treatments, the nude mice bearing intracranial gliomas were euthanized, and their brain tumors were excised, fixed in 4% paraformaldehyde, embedded in paraffin, and sectioned into 10-µm slices. The slices were then blocked for 1 hour with bovine serum albumin (BSA). The sections were incubated overnight at 4°C with an anti-GRP78 antibody (dilution: 1:100), followed by incubation for 2 hours with a FITC-conjugated secondary antibody (dilution: 1:500). Finally, the sections were counterstained with DAPI to highlight the cell nuclei. After mounting the slides, they were examined using a laser confocal microscope.

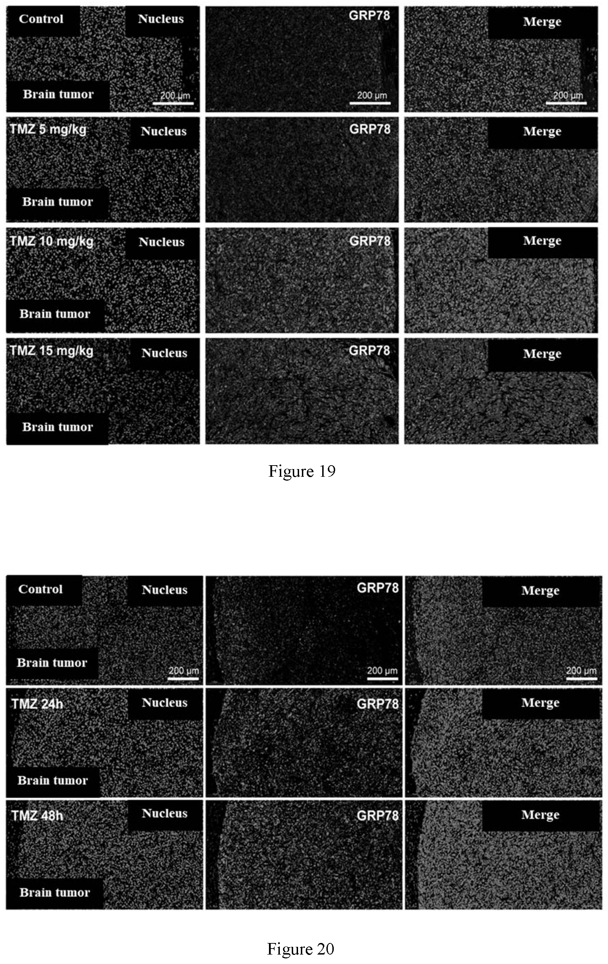

Mice bearing intracranial glioma were administered various doses of TMZ via gavage. After 48 hours, brain tumors were collected and analyzed using the method described above; the results were shown in Figure 19. As illustrated in Figure 19, varying degrees of increased GRP78 protein expression in the tumors were induced by treatment with high-, medium-, and low-concentration TMZ. Considering safety and efficacy comprehensively, a dose of 10 mg/kg was selected for subsequent experiments.

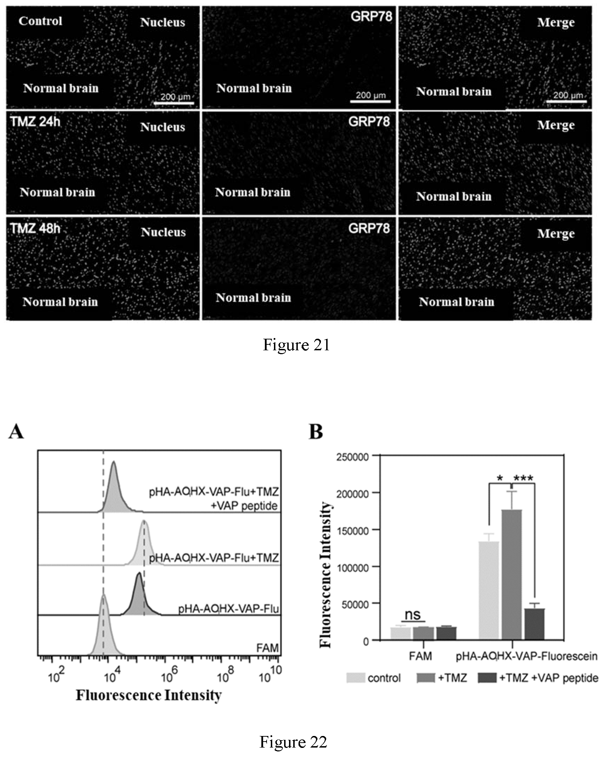

Mice bearing intracranial glioma were administered 10 mg/kg TMZ by gavage. Brain tumors or normal brain tissue samples were collected at different time points and analyzed using the methods described above. The results were shown in Figure 20 and 21. As shown in Figure 20, 48 hours after administration, GRP78 protein expression in the tumor site was significantly increased, consistent with the Western blot results. As shown in Figure 21, GRP78 protein expression in normal brain tissue remained unchanged at different time points following drug administration, indicating that GRP78 protein expression in normal tissue is low and is not upregulated by chemotherapeutic drug.

### Example 10 The uptake of whole-process targeting peptide modified flourescein by U87 cells pre-treated with TMZ

*In vitro* cell uptake assay: Monolayer-cultured cells were collected in the logarithmic growth phase, then digested with 0.25% trypsin. A single-cell suspension, prepared using DMEM medium supplemented with 10% fetal bovine serum, was seeded with 1 × 10⁵ cells per well into a 12-well culture plate, with 1 mL of medium per well. The culture plate was transferred to a CO₂ incubator and culture overnight at 37°C, 5% CO₂, and saturated humidity. Then, the culture medium was replaced with DMEM medium containing 10% fetal bovine serum and 5 µg/mL TMZ. After 48 hours, the culture medium was aspirated from the wells, and FAM and pHA-AO₁HX-VAP-fluorescein solutions was added in the cell wells separately incubating at 37°C for 4 hours. The supernatant was then discarded. The cells were washed three times with PBS solution, and proceed with flow cytometric analysis.

Competition inhibition experiment: To further investigate whether the enhanced uptake of whole-process targeting peptide modified fluorescein following TMZ pre-treatment is mediated via the GRP78 receptor, U87 cells pretreated with TMZ for 48 hours were subjected to a competitive inhibition assay simultaneously. The pretreated cells were dissociated by trypsinization and dispersed at a density of 10⁵ cells/mL in 2-mL EP tubes, pre-cooled at 4°C for 30 minutes. Subsequently, a 100 µM VAP peptide solution prepared in cell culture medium was added, and the cells were incubated at 4°C for 2 hours to allow receptor saturation. Then, pHA-AO₁HX-VAP-fluorescein was added, and the cells were co-incubated at 4°C for another 12 hours. Following three washes with PBS, fresh PBS was added to resuspend the cells, and flow cytometric analysis was performed. The results were shown in Figure 22. As illustrated in Figure 22, 48-hour pretreatment with TMZ significantly enhanced the uptake of pHA-AO₁HX-VAP-modified fluorescein by U87 cells. The fluorescence intensity in the pHA-AO₁HX-VAP-fluorescein group after TMZ pretreatment was 1.35 times that of the control group, closely matching the fold increase in GRP78 protein expression induced by TMZ. After TMZ pretreatment, when an excess amount of VAP was used to saturate the GRP78 receptors on the cell membrane surface, the uptake of pHA-AO₁HX-VAP-modified fluorescein by U87 cells was markedly reduced, confirming that TMZ indeed promotes the uptake of pHA-AO₁HX-VAP-fluorescein by U87 cells through upregulation of GRP78 protein expression.

### Example 11 The uptake of whole-process targeting peptide-drug complex by U87 cells pre-treated with TMZ

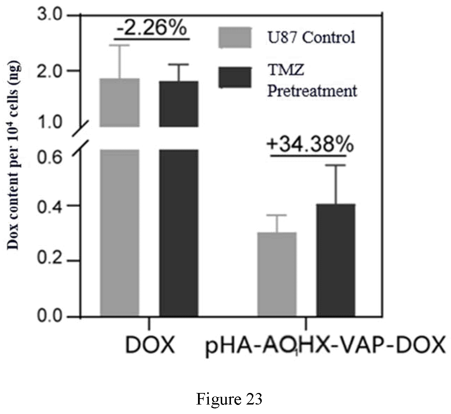

Using the same method, U87 cells were pre-treated with TMZ. After 48 hours, the culture medium in the culture plates was aspirated, and DOX and pHA-AO₁HX-VAP-DOX solutions were added separately. The cells were incubated at 37°C for 4 hours, after which the supernatant was discarded. The cells were then digested and collected, washed twice with PBS, and counted. During the final wash, any remaining supernatant was wholly removed as much as possible. An acidified methanol solution (Vₘₑₜₕₐₙₒₗ : V_{Methanoic acid} = 98:2) was added to the cell pellet, followed by sonication to lyse the cells. After centrifugation at 10,000 rpm for 10 minutes, the supernatant was collected and used to determine the concentration of DOX. The results were shown in Figure 23. As illustrated in Figure 23, pre-treatment with TMZ for 48 hours significantly enhanced the uptake of pHA-AO₁HX-VAP-DOX by U87 cells. After pre-treatment, the average drug amount in the pHA-AO₁HX-VAP-DOX group was 1.34 times that of the control group.

### Example 12 In vivo distribution of - whole-process targeting peptides modified fluorescein following pre-administration of TMZ in a nude mouse model of orthotopic glioma

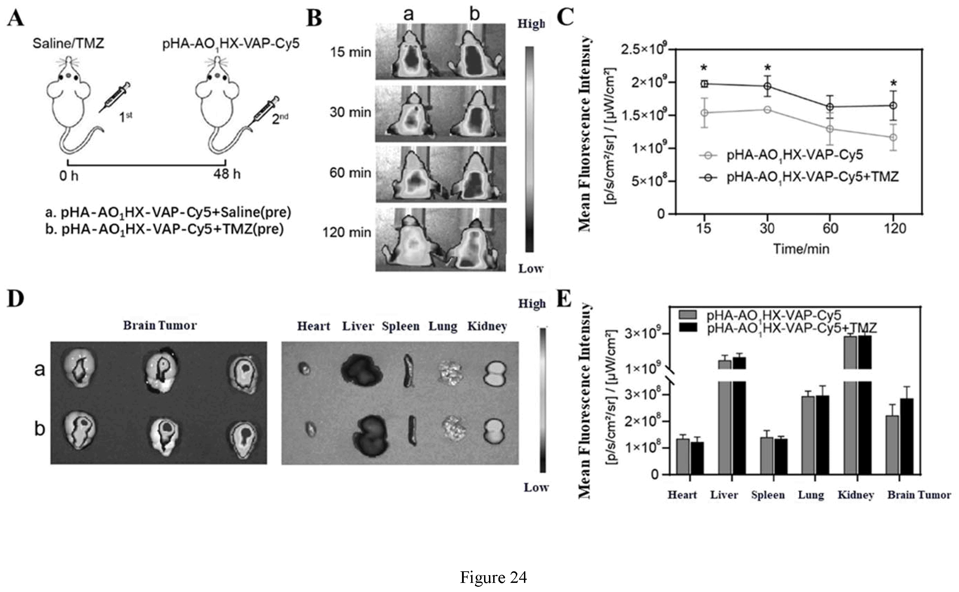

A nude mouse weighing approximately 20 g, was anesthetized and then fixed onto a stereotaxic brain apparatus. Orthotopic glioma mouse model was established by implanting 5 µL of U87 cell suspension (8×10⁵ cells/mL) into the striatum of mice (0.6 mm anteroposterior to the bregma, 1.8 mm lateral to the midline, and 3 mm deep). Ten days after tumor implantation, as shown in A in Figure 24, mice were administered either 10 mg/kg TMZ by gavage or saline via tail vein injection. 48 hours later, mice received an intravenous injection of a pHA-AO₁HX-VAP-Cy5 solution with the same fluorescence intensity. Two hours after the injection, the mice were anesthetized, perfused with saline, and the major organs including the brain, heart, liver, spleen, lungs, and kidneys were collected. Fluorescence distribution in each organ was then visualized using a small-animal *in vivo* imaging system; the results were shown in Figure 24. As illustrated in Figure 24, pre-administration of TMZ significantly enhanced the accumulation of the pHA-AO₁HX-VAP-modified fluorescent in the brain. After 2 hours, the mice were euthanized and perfused, and tissues were harvested for ex vivo imaging. Semi-quantitative analysis revealed that the fluorescence intensity in the TMZ-pre-treated group was 1.21 times higher than that in the group receiving pHA-AO₁HX-VAP-Cy5 alone. Ex vivo organ analysis further demonstrated that pre-administration of TMZ did not alter the distribution of the pHA-AO₁HX-VAP-modified fluorescent in normal organs, suggesting that the combination therapy does not pose any safety risks to peripheral tissues.

### Example 13 Efficacy of TMZ combined with pHA-AO₁HX-VAP-DOX in the treatment of in situ glioma

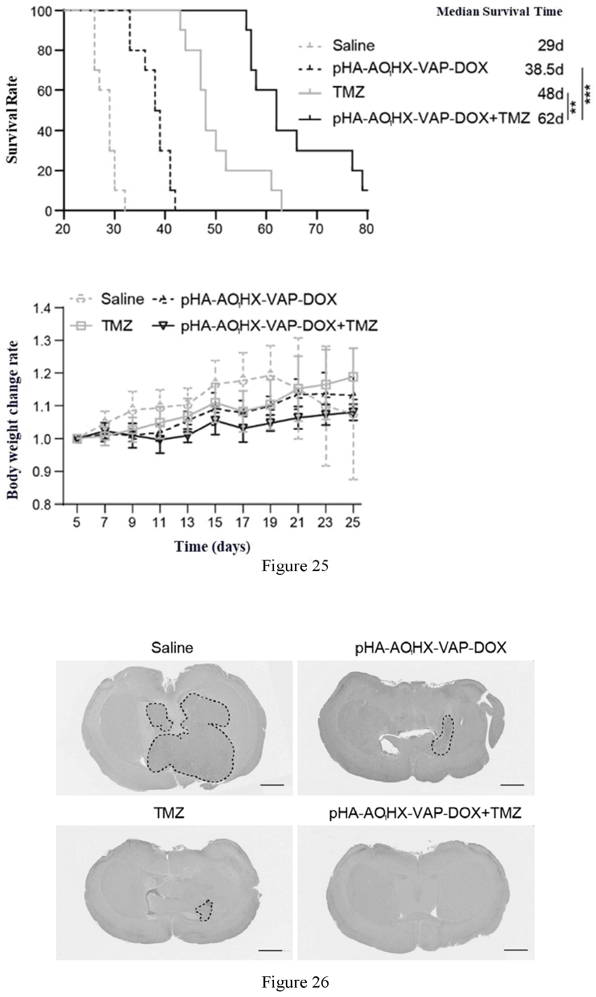

The *in situ* glioma model was established as above, with the day of modeling set as Day 0. On Days 3, 5, 7, 9, 11, 13, 15, 17, 19, and 21, mice were administered TMZ by gavage at a single dose of 10 mg/kg. On Days 5, 7, 9, 11, 13, 15, 17, 19, 21, and 23, pHA-AO₁HX-VAP-DOX was administered to mice via tail vein injection at a single dose equivalent to 3 mg/kg of DOX. At each administration of pHA-AO₁HX-VAP-DOX, changes in mouse body weight were recorded, and the time of death for the model mice was also documented. Survival curves were plotted, and the results were shown in Figure 25. As illustrated in A in Figure 25, the median survival time in the saline group was 29 days, whereas it was 38.5 days in the pHA-AO₁HX-VAP-DOX group and 48 days in the TMZ group. In the group treated with TMZ pre-administration followed by pHA-AO₁HX-VAP-DOX, the median survival time was extended to 62 days, showing a significant difference compared to the groups treated with either drug alone, thus highlighting the advantage of the drug combination therapy.

Synergistic effect between TMZ and pHA-AO₁HX-VAP-DOX in the combination was evaluated using the Jin Zhengjun formula. $q = \frac{{E}_{A + B}}{{E}_{A} + {E}_{B} - {E}_{A} \times {E}_{B}}$

(Wherein, E_{A} and E_{B} represent the effects of drugs A and B when administered individually, while E_{A+B} represents the effect when drugs A and B were used in combination. The median survival extension rate is used for conversion, where the median survival extension rate (%) = (median survival time of mice in the treatment group-median survival time of mice in the control group) / median survival time of mice in the control group × 100%. A q-value between 0.85 and 1.15 indicates an additive effect; a q-value greater than 1.15 indicates a synergistic effect; and a q-value less than 0.85 indicates an antagonistic effect.) The calculated q-value is 1.48, indicating that TMZ exhibits a significantly synergistic effect when combined with pHA-AO₁HX-VAP-DOX.

As shown in B in Figure 25, during the administration period, with the exception of the saline group, whose mice began to experience weight loss starting on day 21 due to tumor progression, no significant weight loss was observed in the other groups, indicating that this drug combination exhibits good safety profile.

On the second day after completion of pHA-AO₁HX-VAP-DOX administration, two nude mice from each group were euthanized, and brain tissues were collected, fixed in 4% paraformaldehyde, dehydrated, cleared, embedded in paraffin, sectioned into wax blocks, dewaxed, stained with hematoxylin and eosin (HE), mounted with coverslips, and examined using a slide scanner. The results were shown in Figure 26. As illustrated in Figure 26, both TMZ and pHA-AO₁HX-VAP-DOX administered alone could partially inhibit the growth of orthotopic gliomas. Moreover, no obvious tumors were detected in the brains of mice treated with TMZ pre-administration combined with pHA-AO₁HX-VAP-DOX, suggesting that this drug combination can significantly suppress the growth of orthotopic gliomas.

## Claims

1. A whole-process targeting peptide comprising pHA and VAP covalently linked via a bridging structure; wherein the VAP is ^{L}VAP, a retro peptide of ^{L}VAP, ^{D}VAP, or ^{S}VAP; wherein, the amino acid sequence of ^{L}VAP is shown as SEQ ID NO: 1; ^{S}VAP is the D-configured peptide of ^{L}VAP; and ^{D}VAP is the D-configuration peptide of a retro peptide of ^{L}VAP;
and the bridging structure is an amino-monoethylene glycol-carboxylic acid or derivatives thereof (AOₙHX), which has the following general formula: 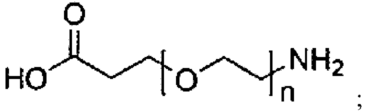
wherein, n is a natural number, preferably ranging from 1 to 20, and more preferably from 1 to 5.

2. The whole-process targeting peptide of claim 1, wherein the VAP is ^{D}VAP, and n is 1, 2, 3, or 5;
preferably, the whole-process targeting peptide is pHA-AOₙHX-^{D}VAP.

3. A targeted diagnostic peptide complex formed by linking the whole-process targeting peptide of claim 1 or claim 2 to an imaging moiety;
preferably, the imaging moiety is an optical imaging moiety, a magnetic resonance imaging agent, and/or a radionuclide imaging agent;
more preferably, the optical imaging moiety is selected from the following groups:
(1) one or more of the fluorescent probe molecules: FITC, FAM, 6-TET, 5-TAMRA, HEX, and 6-JOE;
(2) one or more of the near-infrared dye molecules: Cy3, Cy3.5, Cy5, Cy5.5, Cy7, IR783, IR820, DiR, DiD, BODIPY630/650-X, BODIPY650/665-X, BODIPY665/676, TO-PRO-3, and TO-PRO-5;
(3) one or more of the chemiluminescent substances: luminol, isoluminol, AMPPD, CSPD, CDP-star, luciferin, and Raman probes;
the magnetic resonance imaging agent is a chelate of a Gd-based magnetic resonance substance; and the radionuclide imaging agent is selected from one or more of the chelates of the following imaging radionuclides: ¹⁸F, ³²P, ³⁵S, ⁶⁴Cu, ^{67/68}Ga, ⁷⁵Se, ⁸⁹Zr, ⁸⁶Y, ^{99m}Tc, ^{111/111m}In, ^{123/125}I, ¹⁷⁷Lu, and ^{149/161}Tb;
more preferably, the magnetic resonance imaging agent or radionuclide imaging agent comprises a bifunctional chelator, and a nuclide for magnetic resonance imaging or a radiopharmaceutical nuclide for imaging; wherein the bifunctional chelator is selected from one or more of the following: DOTA, DOTAGA, NOTA, NOTAGA, NODA, DTPA, TETA, CB-TE2A, Cyclam, DFO, MAG3, EC, EDTA, DADT, HYNIC, CE-DTS, and NS3.

4. A targeted therapeutic peptide complex formed by linking the whole-process targeting peptide of claim 1 or claim 2 to a therapeutic drug;
preferably, the whole-process targeting peptide is a pHA-AOₙHX-VAP; and/or, the whole-process targeting peptide linking to or condensing with the therapeutic drug directly or via a pH-sensitive hydrazone bond, or via a pH-sensitive boronate ester bond, or via a disulfide bond.

5. The targeted therapeutic peptide complex of claim 4, wherein the therapeutic drug is selected from one or more of the following: a tumor chemotherapeutic drug, an anti-tumor stem cell drug, a molecular-targeted drug, a peptide drug, an antibody drug, and a therapeutic radionuclide chelate;
preferably, the tumor chemotherapeutic drug is selected from the following groups:
(1) one or more of anthracyclines, such as DOX and epirubicin;
(2) one or more of taxanes, such as paclitaxel, docetaxel, and cabazitaxel;
(3)one or more of camptothecins, such as camptothecin, hydroxycamptothecin, 9-nitrocamptothecin, and irinotecan;
(4) one or more of vinca alkaloids, such as vincristine and vinblastine;
(5) one or more of proteasome inhibitors, such as bortezomib and carfilzomib;
the anti-tumor stem cell drug is selected from lactone-based anti-tumor drugs, such as parthenolide and derivatives thereof;
the molecular-targeted drug is selected from one or more of the following: trametinib, imatinib, nilotinib, dasatinib, everolimus, erlotinib, sunitinib, sorafenib, ibrutinib, regorafenib, vemurafenib, and olaparib;
the peptide drug is a p53-activating peptide;
the antibody drug is selected from one or more of the following: rituximab, bevacizumab, trastuzumab, cetuximab, pertuzumab, ipilimumab, nivolumab, and PD-L1 antibodies, as well as their genetically engineered antibody fragments, including Fab fragments, single-domain antibodies, Fv fragments, single-chain antibodies, bispecific small-molecule antibodies, microantibodies, and nanobodies;
and the therapeutic radionuclide chelate is selected from one or more of the chelates of the following therapeutic radionuclides of ⁹⁰Y, ¹³¹I, ^{152/155}Tb, ¹⁵³Sm, ¹⁷⁷Lu, ^{186/188}Re, ²¹¹At, ^{212/213}Bi, ²¹²Pb, ²²⁵Ac, and ²²⁷Th;
more preferably, the therapeutic radionuclide chelate comprises a bifunctional chelator and a therapeutic radionuclide, wherein the bifunctional chelator is selected from one or more of the following: DOTA, DOTAGA, NOTA, NOTAGA, NODA, DTPA, TETA, CB-TE2A, Cyclam, DFO, MAG3, EC, EDTA, DADT, HYNIC, CE-DTS, and NS3;
furthermore preferably, the targeted therapeutic peptide complex is selected from:
(1) pHA-AOₙHX-VAP-DOX;
(2) pHA-AOₙHX-VAP-Paclitaxel;
(3) pHA-AOₙHX-VAP-Bortezomib;
(4) pHA-AOₙHX-VAP-PMI;
(5) pHA-AOₙHX-VAP-αPDL1;
(6) pHA-AOₙHX-VAP-DOTA-Gd.

6. A targeted peptide-polymer carrier material formed by linking the whole-process targeting peptide of claim 1 or claim 2 to a polyethylene glycol-complex; wherein the complex is a lipophilic material moiety or a hydrophilic ligand moiety;
preferably, the lipophilic material moiety is selected from one or more of the following: phospholipids, polylactic acid, poly(lactide-co-glycolide), and polycaprolactone; the hydrophilic ligand moiety is biotin.

7. A targeted drug delivery system comprising the targeted peptide-polymer carrier material of claim 6 and excipients;
when the polyethylene glycol-complex in the targeted peptide-polymeric carrier material is polyethylene glycol-phospholipid, the targeted drug delivery system can be a liposome delivery system, a micell delivery system, or a disc-shaped delivery system;
when the polyethylene glycol-complex is polyethylene glycol-polylactic acid, polyethylene glycol-poly(lactide-co-glycolide), or polyethylene glycol-polycaprolactone, the targeted drug delivery system is a micell delivery system or a nanoparticle delivery system;
when the polyethylene glycol-complex is polyethylene glycol-biotin, the targeted drug delivery system is a biomembrane-coated nano delivery system;
preferably, the excipient comprises one or more of cholesterol, phospholipids, polyethylene glycol-phospholipids, polyethylene glycol-polylactic acid, polyethylene glycol-poly(lactide-co-glycolide), and polyethylene glycol-polycaprolactone.

8. The targeted drug delivery system of claim 7, wherein the targeted drug delivery system is loaded with a drug, and the drug is a diagnostic drug or a therapeutic drug;
preferably, the diagnostic drug is the imaging moiety as defined in the targeted diagnostic peptide complex of claim 3; and the therapeutic drug is the therapeutic drug as defined in the targeted therapeutic peptide complex of claim 5.

9. A drug combination for combined therapy, comprising a chemotherapeutic drug, and the targeted therapeutic peptide complex of claim 4 or claim 5 or the targeted drug delivery system of claim 7 or claim 8;
preferably, the chemotherapeutic drug is selected from one or more of the following: DOX, epirubicin, TMZ, paclitaxel, docetaxel, cabazitaxel, camptothecin, hydroxycamptothecin, 9-nitrocamptothecin, irinotecan, vinblastine, bortezomib, carfilzomib, and 5-fluorouracil;
more preferably, the administration regimen for the drug combination involves first administering a chemotherapeutic drug, followed by administering the targeted therapeutic peptide complex of claim 4 or claim 5, or the targeted drug delivery system of claim 7 or claim 8 after a certain period of time; wherein the certain period of time is preferably 24-48 hours;
furthermore preferably, the chemotherapeutic drug is TMZ, and/or the targeted therapeutic peptide complex is pHA-AOₙHX-VAP-DOX; more preferably, n is 1 or 2.

10. A use of the whole-process targeting peptide of claim 1 or claim 2, the targeted diagnostic peptide complex of claim 3, the targeted therapeutic peptide complex of claim 4 or claim 5, the targeted peptide-polymer carrier material of claim 6, the targeted drug delivery system of claim 7 or claim 8, and/or the drug combination of claim 9, in the preparation of imaging agent, tracer agent, and/or targeted therapeutic drug for a tumor such as a brain tumor or a peripheral tumor.

11. The whole-process targeting peptide of claim 1 or claim 2, the targeted diagnostic peptide complex of claim 3, the targeted therapeutic peptide complex of claim 4 or claim 5, the targeted peptide-polymer carrier material of claim 6, the targeted drug delivery system of claim 7 or claim 8, and/or the drug combination of claim 9, for use in the diagnosis and/or treatment of a tumor;
preferably, the tumor is a brain tumor or a peripheral tumor.

12. A method for diagnosing and/or treating a tumor, comprising administering to a subject in need a diagnostically effective dose or a therapeutically effective dose of the targeted therapeutic peptide complex of claim 4 or claim 5, the targeted drug delivery system of claim 7 or claim 8, and/or the drug combination of claim 9;
preferably, the tumor is a brain tumor or a peripheral tumor.
